# EUROPEAN PATENT SPECIFICATION

(11) **EP 2 629 690 B1**
(45) Date of publication and mention of the grant of the patent: **31.07.2019**
(21) Application number: 11778752.3
(22) Date of filing: 20.10.2011
(51) Int. Cl.: A61B 18/14, A61B 18/08, A61N 7/02, A61B 18/24, A61B 18/00, A61B 18/18, A61N 7/00

(54) **CATHETER APPARATUSES HAVING EXPANDABLE MESH STRUCTURES FOR RENAL NEUROMODULATION**
KATHETERVORRICHTUNGEN MIT EXPANDIERBAREN NETZSTRUKTUREN FÜR NIERENNERVENMODULATION
APPAREILS DE CATHÉTER AYANT DES STRUCTURES DE MAILLE DILATABLES POUR NEUROMODULATION RÉNALE

(30) Priority: 20.10.2010 US 405117 P
(43) Date of publication of application: 28.08.2013
(73) Proprietor: Medtronic Ardian Luxembourg S.à.r.l., 2124 Luxembourg (LU)
(72) Inventor: BEETEL, Robert J., Mountain View, CA 94043 (US); GRISWOLD, Erik, Penngrove, CA 94951 (US); ZARINS, Denise, Saratoga, CA 95070 (US); ABOYTES, Maria G., Palo Alto, CA 94303 (US)
(74) Representative: Zimmermann & Partner Patentanwälte mbB
(86) International application number: PCT/US2011/057153
(87) International publication number: WO 2012/054762

(56) References cited:
- EP-A2- 2 213 257
- WO-A1-2012/015720
- WO-A2-2006/041881
- US-A- 5 226 430

## Description

### TECHNICAL FIELD

The present technology relates generally to renal neuromodulation. In particular, several embodiments are directed to catheter apparatuses having expandable mesh structures for intravascular renal neuromodulation.

### BACKGROUND

The sympathetic nervous system (SNS) is a primarily involuntary bodily control system typically associated with stress responses. Fibers of the SNS innervate tissue in almost every organ system of the human body and can affect characteristics such as pupil diameter, gut motility, and urinary output. Such regulation can have adaptive utility in maintaining homeostasis or in preparing the body for rapid response to environmental factors. Chronic activation of the SNS, however, is a common maladaptive response that can drive the progression of many disease states. Excessive activation of the renal SNS in particular has been identified experimentally and in humans as a likely contributor to the complex pathophysiology of hypertension, states of volume overload (such as heart failure), and progressive renal disease. For example, radiotracer dilution has demonstrated increased renal norepinephrine (NE) spillover rates in patients with essential hypertension.

Cardio-renal sympathetic nerve hyperactivity can be particularly pronounced in patients with heart failure. For example, an exaggerated NE overflow from the heart and kidneys to plasma is often found in these patients. Heightened SNS activation commonly characterizes both chronic and end stage renal disease. In patients with end stage renal disease, NE plasma levels above the median have been demonstrated to be predictive for both all causes of death and death from cardiovascular disease. This is also true for patients suffering from diabetic or induced contrast nephropathy. Evidence suggests that sensory afferent signals originating from diseased kidneys are major contributors to initiating and sustaining elevated central sympathetic outflow.

Sympathetic nerves innervating the kidneys terminate in the blood vessels, the juxtaglomerular apparatus, and the renal tubules. Stimulation of the renal sympathetic nerves can cause increased renin release, increased sodium (Na⁺) reabsorption, and a reduction of renal blood flow. These neural regulation components of renal function are considerably stimulated in disease states characterized by heightened sympathetic tone and likely contribute to increased blood pressure in hypertensive patients. The reduction of renal blood flow and glomerular filtration rate as a result of renal sympathetic efferent stimulation is likely a cornerstone of the loss of renal function in cardio-renal syndrome (i.e., renal dysfunction as a progressive complication of chronic heart failure). Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin II and aldosterone activation consequent to renin release), and diuretics (intended to counter the renal sympathetic mediated sodium and water retention). These pharmacologic strategies, however, have significant limitations including limited efficacy, compliance issues, side effects, and others. Accordingly, there is a strong public-health need for alternative treatment strategies.

WO-A-2006/041 881 discloses a catheter for renal neuromodulation.

EP-A-2 213 257 discloses a braided mesh catheter for electrophysiology procedures.

### SUMMARY OF THE INVENTION

The invention provides a catheter apparatus according to claim 1. Preferred embodiments are defined in the dependent claims. The methods disclosed herein, as well as all other embodiments, aspects and examples herein that do not fall under the scope of claim 1, are not claimed, but are useful for understanding the invention.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
Figure 1 illustrates an intravascular renal neuromodulation system configured in accordance with an embodiment of the present technology.
Figure 2 illustrates modulating renal nerves with a catheter apparatus having an expandable mesh structure in accordance with an embodiment of the present technology.
Figure 3 is a view of a distal portion of a shaft and a mesh structure in a delivery state (e.g., a low-profile or collapsed configuration) used in conjunction with a guide catheter within a renal artery in accordance with an embodiment of the present technology.
Figure 4 is a view of the distal portion of the shaft and the mesh structure of Figure 3 in a deployed state (e.g., expanded configuration) within a renal artery in accordance with an embodiment of the technology.
Figure 5 is a cross-sectional view along line 5-5 of Figure 4 of the mesh structure in the expanded configuration inside a patient in accordance with an embodiment of the technology.
Figure 6 is a side perspective view of an embodiment of a distal portion of a shaft and a mesh structure in a collapsed configuration in accordance with an embodiment of the technology.
Figure 7 is a side perspective view of the mesh structure of Figure 6 in an expanded configuration outside a patient in accordance with an embodiment of the technology.
Figure 8 is a view of a treatment device within a renal artery used in conjunction with a guide catheter in accordance with an embodiment of the technology.
Figure 9 is a view of a treatment device within a renal artery with an open-ended basket configuration in accordance with an embodiment of the technology.
Figure 10 is a view of a treatment device within a renal artery used in conjunction with a guide wire in accordance with an embodiment of the technology.
Figure 11 is a view of a treatment device within a renal artery with a close-ended basket configuration in accordance with an embodiment of the technology.
Figure 12A is a view of a treatment device within a renal artery with an open-ended tubular mesh configuration in accordance with an embodiment of the technology.
Figure 12B is a view of a treatment device within a renal artery with another open-ended tubular mesh configuration in accordance with an embodiment of the technology.
Figure 13 is a view of a treatment device within a renal artery with multiple energy delivery elements associated with the structure in accordance with an embodiment of the technology.
Figure 14 is a partial side perspective view of a mesh structure with energy delivery leads threaded through the mesh in accordance with an embodiment of the technology.
Figure 15 is a partial side perspective view of a mesh structure with an energy delivery element threaded onto the fibers of the mesh in accordance with an embodiment of the technology.
Figure 16 is a partial side perspective view of a mesh structure with a ribbon electrode energy delivery element wound about the mesh in accordance with an embodiment of the technology.
Figure 17 is a partial side perspective view of a mesh structure with an insulated ribbon electrode having exposed areas for energy delivery in accordance with an embodiment of the technology.
Figure 18 is a side perspective view of a mesh structure in a collapsed configuration with multiple energy delivery elements distributed about the mesh structure in accordance with an embodiment of the technology.
Figure 19 is a view of the mesh structure of Figure 18 in an expanded configuration within a renal artery showing axial distances between the energy delivery elements in accordance with an embodiment of the technology.
Figure 20A is an end view of a mesh structure in an expanded configuration showing the circumferential offset of the energy delivery elements in accordance with an embodiment of the technology.
Figure 20B is a side view of the mesh structure of Figure 20A.
Figure 20C is an end view of a mesh structure in an expanded configuration showing the circumferential offset of the energy delivery elements in accordance with an embodiment of the technology.
Figure 20D is a side view of the mesh structure of Figure 20C.
Figure 21 is a view of a shaped energy delivery element in accordance with an embodiment of the technology.
Figure 22 is a view of an alternative shaped energy delivery element in accordance with an embodiment of the technology.
Figure 23 is a side perspective view of a mesh structure with varying braid pitch in adjacent cylindrical sections in accordance with an embodiment of the technology.
Figure 24 is a side perspective view of a mesh structure with varying braid pitch in an area surrounding an energy delivery element in accordance with an embodiment of the technology.
Figure 25 is a side perspective view of a mesh structure in which the mesh structure is electrically conductive and serves as the energy delivery element in accordance with an embodiment of the technology.
Figure 26 is a side perspective view of a mesh structure of a treatment device in which the mesh structure is electrically conductive and in which uninsulated areas of the mesh structure serve as the energy delivery elements in accordance with an embodiment of the technology.
Figure 27 is a side perspective view of a treatment device including multiple mesh structures that are electrically conductive and that include uninsulated areas of the mesh structure that are electrically isolated between the two mesh structures in accordance with an embodiment of the technology.
Figure 28 is a view of a mesh structure having a varying circumferential shape in accordance with an embodiment of the technology.
Figure 29 is a view of a mesh structure having a varying circumferential shape in accordance with an embodiment of the technology.
Figure 30 is a side perspective view of a mesh structure of a treatment device in which portions of electrically conductive mesh are separated by portions of electrically nonconductive mesh in accordance with an embodiment of the technology.
Figure 31 is a side perspective view of an alternative mesh structure of a treatment device in which portions of electrically conductive mesh are separated by portions of electrically nonconductive mesh in accordance with an embodiment of the technology.
Figure 32 is a side perspective view of an alternative mesh structure of a treatment device in which portions of electrically conductive mesh are separated by portions of electrically nonconductive mesh in accordance with an embodiment of the technology.
Figure 33 is an illustration of blood flow in a renal artery in accordance with an embodiment of the technology.
Figure 34 is a view of an embodiment of a treatment device in a renal artery, the treatment device including a fluid redirecting element within a mesh structure in accordance with an embodiment of the technology.
Figure 35 is a side perspective view of a mesh structure with a fluid redirecting element in which the mesh structure is in an expanded configuration in accordance with an embodiment of the technology.
Figure 36 is a side perspective view of a mesh structure with a fluid redirecting element in which the mesh structure is in a collapsed configuration in accordance with an embodiment of the technology.
Figure 37 is a partially cutaway side view of a grooved fluid redirecting element within a mesh structure in accordance with an embodiment of the technology.
Figure 38 is a partially cutaway side view of a porous fluid redirecting element within a mesh structure in accordance with an embodiment of the technology.
Figure 39 is a partially cutaway side view of a mesh fluid redirecting element within a mesh structure in accordance with an embodiment of the technology.
Figure 40 is a view of a fluid redirecting element in conjunction with a parachute-type mesh structure in accordance with an embodiment of the technology.
Figure 41 is a graph depicting an energy delivery algorithm that may be used in conjunction with the system of Figure 1 in accordance with an embodiment of the technology.
Figure 42 is a kit for packaging components of the system of Figure 1 in accordance with an embodiment of the technology.
Figure 43 is a conceptual illustration of the sympathetic nervous system (SNS) and how the brain communicates with the body via the SNS.
Figure 44 is an enlarged anatomic view of nerves innervating a left kidney to form the renal plexus surrounding the left renal artery.
Figures 45A and 45B provide anatomic and conceptual views of a human body, respectively, depicting neural efferent and afferent communication between the brain and kidneys.
Figures 46A and 46B are, respectively, anatomic views of the arterial and venous vasculatures of a human.

### DETAILED DESCRIPTION

The present technology is directed to apparatuses, systems, and methods for achieving electrically- and/or thermally-induced renal neuromodulation (i.e., rendering neural fibers that innervate the kidney inert or inactive or otherwise completely or partially reduced in function) by percutaneous transluminal intravascular access. In particular, embodiments of the present technology relate to apparatuses, systems, and methods that incorporate a catheter treatment device having an expandable mesh structure or other open structure. The expandable mesh structure can include and/or is associated with at least one element configured to deliver energy (e.g., electrical energy, radiofrequency electrical energy, pulsed electrical energy, thermal energy) to a renal artery after being advanced via a catheter along a percutaneous transluminal path (e.g., a femoral artery puncture, an iliac artery and the aorta, a transradial approach, or another suitable intravascular path). The expandable mesh structure is sized and shaped so that the energy delivery element contacts an interior wall of the renal artery when the mesh structure is in an expanded configuration within the renal artery. In addition, the mesh portion of the expandable mesh structure allows blood to flow through the mesh, thereby maintaining blood flow to the kidney. Further, blood flow in and around the mesh structure may cool the associated energy delivery element and/or surrounding tissue. In some embodiments, cooling the energy delivery element allows for the delivery of higher power levels at lower temperatures than may be reached without cooling. This feature is expected to help create deeper and/or larger lesions during therapy, reduce intimal surface temperature, and/or allow longer activation times with reduced risk of overheating during treatment.

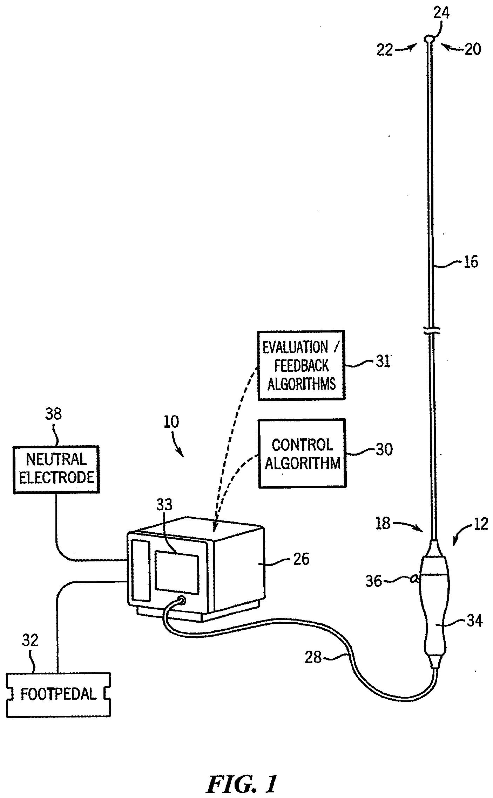

Specific details of several embodiments of the technology are described below with reference to Figures 1-46B. Although many of the embodiments are described below with respect to devices, systems, and methods for intravascular modulation of renal nerves using mesh catheter apparatuses, other applications and other embodiments in addition to those described herein are within the scope of the technology. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described below with reference to Figures 1-46B.

As used herein, the terms "distal" and "proximal" define a position or direction with respect to the treating clinician or clinician's control device (e.g., a handle assembly). "Distal" or "distally" are a position distant from or in a direction away from the clinician or clinician's control device. "Proximal" and "proximally" are a position near or in a direction toward the clinician or clinician's control device.

### I. Renal Neuromodulation

Renal neuromodulation is the partial or complete incapacitation or other effective disruption of nerves innervating the kidneys. In particular, renal neuromodulation comprises inhibiting, reducing, and/or blocking neural communication along neural fibers (i.e., efferent and/or afferent nerve fibers) innervating the kidneys. Such incapacitation can be long-term (e.g., permanent or for periods of months, years, or decades) or short-term (e.g., for periods of minutes, hours, days, or weeks). Renal neuromodulation is expected to efficaciously treat several clinical conditions characterized by increased overall sympathetic activity, and in particular conditions associated with central sympathetic over stimulation such as hypertension, heart failure, acute myocardial infarction, metabolic syndrome, insulin resistance, diabetes, left ventricular hypertrophy, chronic and end stage renal disease, inappropriate fluid retention in heart failure, cardio-renal syndrome, and sudden death. The reduction of afferent neural signals contributes to the systemic reduction of sympathetic tone/drive, and renal neuromodulation is expected to be useful in treating several conditions associated with systemic sympathetic over activity or hyperactivity. Renal neuromodulation can potentially benefit a variety of organs and bodily structures innervated by sympathetic nerves. For example, a reduction in central sympathetic drive may reduce insulin resistance that afflicts patients with metabolic syndrome and Type II diabetics. Additionally, osteoporosis can be sympathetically activated and might benefit from the downregulation of sympathetic drive that accompanies renal neuromodulation. A more detailed description of pertinent patient anatomy and physiology is provided in Section VI below.

Various techniques can be used to partially or completely incapacitate neural pathways, such as those innervating the kidney. The purposeful application of energy (e.g., electrical energy, thermal energy) to tissue by energy delivery element(s) can induce one or more desired thermal heating effects on localized regions of the renal artery and adjacent regions of the renal plexus RP, which lay intimately within or adjacent to the adventitia of the renal artery. The purposeful application of the thermal heating effects can achieve neuromodulation along all or a portion of the renal plexus RP.

The thermal heating effects can include both thermal ablation and non-ablative thermal alteration or damage (e.g., via sustained heating and/or resistive heating). Desired thermal heating effects may include raising the temperature of target neural fibers above a desired threshold to achieve non-ablative thermal alteration, or above a higher temperature to achieve ablative thermal alteration. For example, the target temperature can be above body temperature (e.g., approximately 37°C) but less than about 45°C for non-ablative thermal alteration, or the target temperature can be about 45°C or higher for the ablative thermal alteration.

More specifically, exposure to thermal energy (heat) in excess of a body temperature of about 37°C, but below a temperature of about 45°C, may induce thermal alteration via moderate heating of the target neural fibers or of vascular structures that perfuse the target fibers. In cases where vascular structures are affected, the target neural fibers are denied perfusion resulting in necrosis of the neural tissue. For example, this may induce non-ablative thermal alteration in the fibers or structures. Exposure to heat above a temperature of about 45°C, or above about 60°C, may induce thermal alteration via substantial heating of the fibers or structures. For example, such higher temperatures may thermally ablate the target neural fibers or the vascular structures. In some patients, it may be desirable to achieve temperatures that thermally ablate the target neural fibers or the vascular structures, but that are less than about 90°C, or less than about 85°C, or less than about 80°C, and/or less than about 75°C. Regardless of the type of heat exposure utilized to induce the thermal neuromodulation, a reduction in renal sympathetic nerve activity ("RSNA") is expected.

### II. Selected Embodiments of Renal Neuromodulation Devices Having Mesh Structures

Figure 1 illustrates a renal neuromodulation system 10 ("system 10") configured in accordance with an embodiment of the present technology. The system 10 includes an intravascular treatment device 12 operably coupled to an energy source or energy generator 26. In the embodiment shown in Figure 1, the treatment device 12 (e.g., a catheter) includes an elongated shaft 16 having a proximal portion 18, a handle assembly 34 at a proximal region of the proximal portion 18, and a distal portion 20 extending distally relative to the proximal portion 18. The treatment device 12 further includes an expandable mesh structure 22 including an energy delivery element 24 disposed at or near the distal portion 20 of the shaft 16. As explained in further detail below, the mesh structure 22 is configured to be delivered to a renal blood vessel (e.g., renal artery) in a low-profile or delivery configuration. Upon delivery to the target treatment site within the renal blood vessel, the mesh structure 22 is further configured to be deployed into an expanded or treatment configuration, bringing the energy delivery element 24 in contact with the walls of the vessel. The energy delivery element 24 is configured to deliver energy at the treatment site and provide therapeutically-effective electrically- and/or thermally-induced renal neuromodulation. In some embodiments, the mesh structure 22 may be placed in the deployed configuration or arrangement via remote actuation, e.g., via an actuator 36, such as a knob, pin, or lever carried by the handle 34. In other embodiments, however, the mesh structure 22 may be movable between the delivery and deployed configurations using other suitable mechanisms or techniques (e.g., self-expanding).

As will be described in greater detail below, the energy delivery element 24 is associated with the mesh structure 22. That is, the energy delivery element 24 may be proximate to, adjacent to, adhered to, woven into, or otherwise coupled to the mesh structure 22. The associated energy delivery element 24 may also be formed by selected portions of, or the entirety of, the mesh structure 22 itself. For example, the fibers of the mesh may be capable of delivering energy. It should also be understood that mesh structure 22 may include a plurality of energy delivery elements 24. When multiple energy delivery elements 24 are provided, the energy delivery elements 24 may deliver power independently (i.e., may be used in a monopolar fashion), either simultaneously, selectively, or sequentially, and/or may deliver power between any desired combination of the elements (i.e., may be used in a bipolar fashion). Furthermore, the clinician optionally may be permitted to choose which energy delivery element(s) 24 are used for power delivery in order to form highly customized lesion(s) within the renal artery, as desired. The energy delivery element 24 is mounted or integrated into the mesh structure 22. As the mesh structure is expanded, the energy delivery element is placed in contact with the wall of a renal artery. The mesh structure 22 ensures the contact force of the energy delivery element does not exceed a maximum force, thus advantageously providing a more consistent contact force that may allow for more consistent lesion formation.

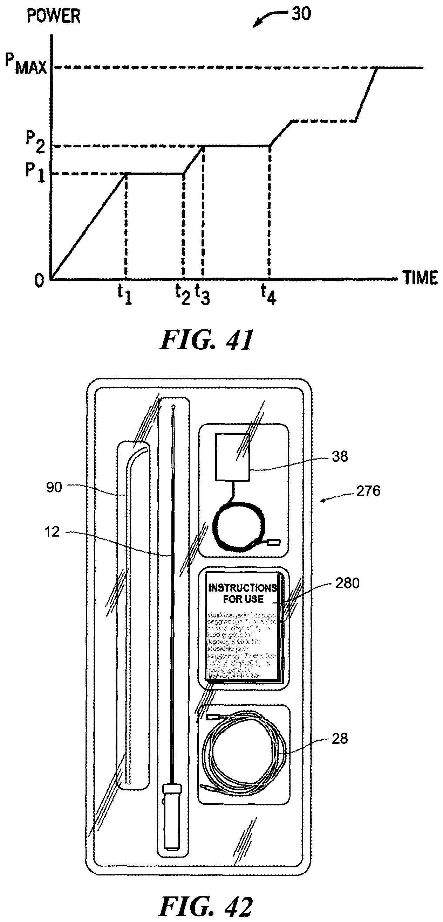

The energy source or energy generator 26 (e.g., a RF energy generator) is configured to generate a selected form and magnitude of energy for delivery to the target treatment site via the energy delivery element 24. The energy generator 26 can be electrically coupled to the treatment device 12 via a cable 28. At least one supply wire (not shown) passes along the elongated shaft 16 or through a lumen in the elongated shaft 16 to the energy delivery element 24 and transmits the treatment energy to the energy delivery element 24. A control mechanism, such as foot pedal 32, may be connected (e.g., pneumatically connected or electrically connected) to the energy generator 26 to allow the operator to initiate, terminate and, optionally, adjust various operational characteristics of the energy generator, including, but not limited to, power delivery. The energy generator 26 can be configured to deliver the treatment energy via an automated control algorithm 30 and/or under the control of the clinician. In addition, the energy generator 26 may include one or more evaluation or feedback algorithms 31 to provide feedback to the clinician before, during, and/or after therapy. Further details regarding suitable control algorithms and evaluation/feedback algorithms are described below with reference to Figures 41-48B.

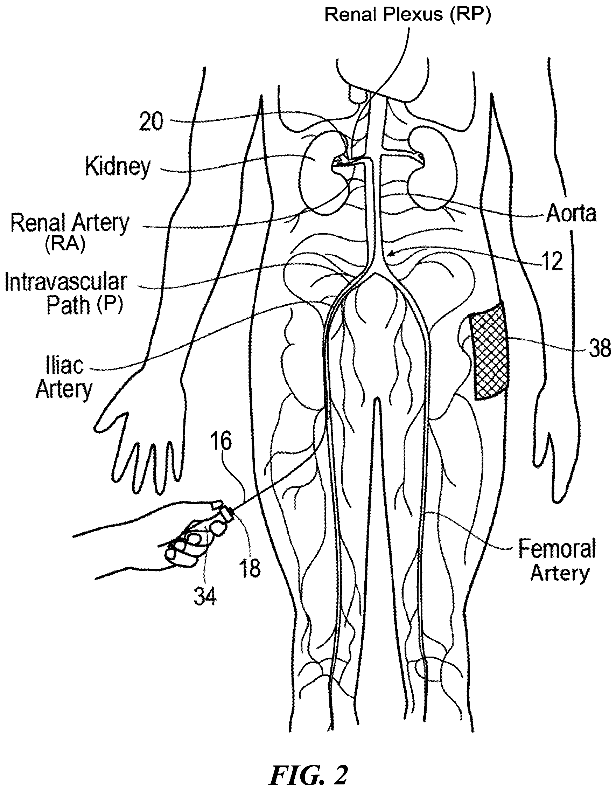

In some embodiments, the system 10 may be configured to provide delivery of a monopolar electric field via the energy delivery elements 24. In such embodiments, a neutral or dispersive electrode 38 may be electrically connected to the energy generator 26 and attached to the exterior of the patient, as shown in Figure 2. Additionally, one or more sensors (not shown), such as one or more temperature (e.g., thermocouple, thermistor, etc.), impedance, pressure, optical, flow, chemical or other sensors, may be located proximate to or within the energy delivery element 24 and connected to one or more supply wires (not shown). For example, a total of two supply wires may be included, in which both wires could transmit the signal from the sensor and one wire could serve dual purpose and also convey the energy to the energy delivery element 24. Alternatively, both wires could transmit energy to the energy delivery element 24.

The generator 26 may be part of a device or monitor that may include processing circuitry, such as a microprocessor, and a display. The processing circuitry may be configured to execute stored instructions relating to the control algorithm 30. The monitor may be configured to communicate with the treatment device 12 (e.g., via cable 28) to control power to the energy delivery element 24 and/or to obtain signals from the energy delivery element 24 or any associated sensors. The monitor may be configured to provide indications of power levels or sensor data, such as audio, visual or other indications, or may be configured to communicate the information to another device.

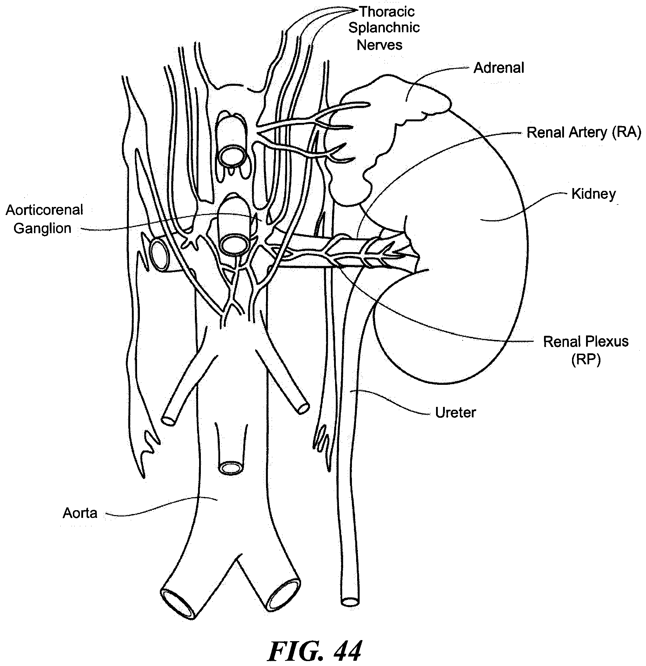

Figure 2 illustrates modulating renal nerves with an embodiment of the system 10, with further detail regarding the relevant anatomy provided below with reference to Figure 44. The treatment device 12 provides access to the renal plexus RP through an intravascular path (P), such as from a percutaneous access site in the femoral (illustrated), brachial, radial, or auxiliary artery to a targeted treatment site within a respective renal artery RA. As illustrated, a section of the proximal portion 18 of the shaft 16 is exposed externally of the patient. By manipulating the proximal portion 18 of the shaft 16 from outside the intravascular path P (e.g., via the handle assembly 34), the clinician may advance the shaft 16 through the sometimes tortuous intravascular path P and remotely manipulate or actuate the distal portion 20 of the shaft 16. Image guidance, e.g., computed tomography (CT), fluoroscopy, intravascular ultrasound (IVUS), optical coherence tomography (OCT), or another suitable guidance modality, or combinations thereof, may be used to aid the clinician's manipulation. Further, in some embodiments image guidance components (e.g., IVUS, OCT) may be incorporated into the treatment device 12 itself. After the mesh structure 22 is adequately positioned in the renal artery RA, it can be expanded or otherwise deployed using the handle 34 or other suitable means until the energy delivery element 24 is in stable contact with the inner wall of the renal artery RA. The purposeful application of energy from the energy delivery element 24 is then applied to tissue to induce one or more desired neuromodulating effects on localized regions of the renal artery and adjacent regions of the renal plexus RP, which lay intimately within, adjacent to, or in close proximity to the adventitia of the renal artery RA. The purposeful application of the incapacitating energy may achieve neuromodulation along all or at least a portion of the renal plexus RP.

The neuromodulating effects are generally a function of, at least in part, power, time, contact between the energy delivery element 24 carried by the mesh structure 22 and the vessel wall, and blood flow through the vessel. The neuromodulating effects may include denervation, thermal ablation, and non-ablative thermal alteration or damage (e.g., via sustained heating and/or resistive heating. Desired thermal heating effects may include raising the temperature of target neural fibers above a desired threshold to achieve non-ablative thermal alteration, or above a higher temperature to achieve ablative thermal alteration. For example, the target temperature may be above body temperature (e.g., approximately 37°C) but less than about 45°C for non-ablative thermal alteration, or the target temperature may be about 45°C or higher for the ablative thermal alteration. Desired non-thermal neuromodulation effects may also further include altering the electrical signals transmitted in a nerve.

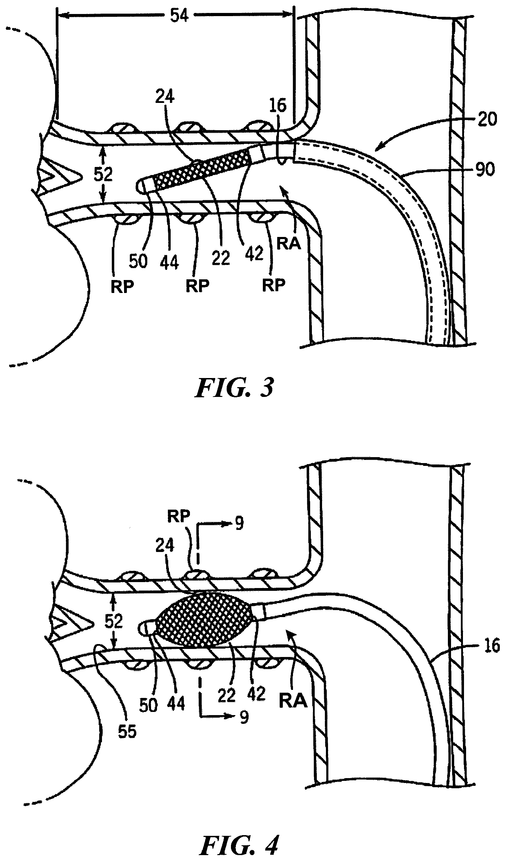

Figure 3 is a cross-sectional view illustrating one embodiment of the distal portion 20 of the shaft 16 and the mesh structure 22 in a delivery state (e.g., low-profile or collapsed configuration) within a renal artery RA, and Figure 4 is a cross-sectional view of the mesh structure 22 in a deployed state (e.g., expanded configuration) within the renal artery RA. Referring first to Figure 3, the collapsed or delivery configuration of the mesh structure 22 facilitates insertion and/or removal of the treatment device 12 and, in certain embodiments, repositioning of the mesh structure 22 within the renal artery RA. In the collapsed configuration, the mesh structure 22 is sized and shaped to fit within the renal artery RA and has a diameter that is less than a renal artery lumen diameter 52 and a length (from a proximal end 42 of the mesh structure 22 to a distal end 44 of the mesh structure 22) that is less than a renal artery length 54.

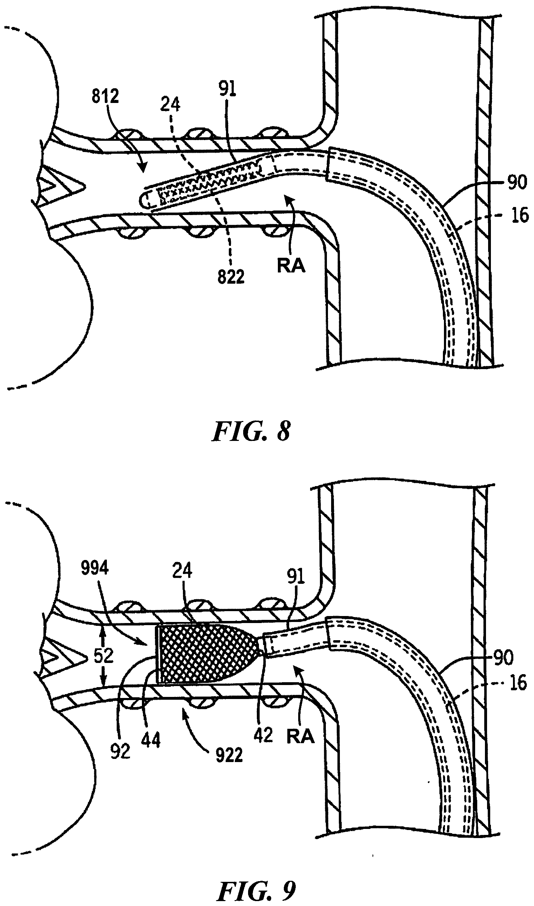

As shown in Figure 3, the distal portion 20 of the shaft 16 may flex in a substantial fashion to gain entrance into a respective left/right renal artery by following a path defined by a guide catheter, a guide wire, or a sheath. For example, the flexing of the distal portion 20 may be imparted by a guide catheter 90, such as a renal guide catheter with a preformed bend near the distal end that directs the shaft 16 along a desired path from the percutaneous insertion site to the renal artery RA. In another embodiment, the treatment device 12 may be directed to the treatment site within the renal artery RA by engaging and tracking a guide wire (not shown) that is inserted into the renal artery RA and extends to the percutaneous access site. In operation, the guide wire is preferably first delivered into the renal artery RA and the elongated shaft 16 comprising a guide wire lumen is then passed over the guide wire into the renal artery RA. In some guide wire procedures, a tubular delivery sheath 91 (described in greater detail below with reference to Figure 8) is passed over the guide wire (i.e., the lumen defined by the delivery sheath slides over the guide wire) into the renal artery RA. Once the delivery sheath 91 (Figure 8) is placed in the renal artery, the guide wire may be removed and exchanged for a treatment catheter (e.g., treatment device 12) that may be delivered through the delivery sheath 91 (Figure 8) into the renal artery RA. Furthermore, in particular embodiments, the flexing may be controlled from the handle assembly 34 (Figures 1 and 2), for example, by the actuatable element 36 or by another control element. In particular, the flexing of the elongated shaft 16 may be accomplished as provided in U.S. Patent Application No. 12/545,648, "Apparatus, Systems, and Methods for Achieving Intravascular, Thermally-Induced Renal Neuromodulation" to Wu et al. Alternatively, or in addition, the treatment device 12 and its distal portion 20 may be flexed by being inserted through a steerable guide catheter (not shown) that includes a preformed or steerable bend near its distal end that can be adjusted or re-shaped by manipulation from the proximal end of the guide catheter.

After locating the mesh structure 22 in the renal artery RA, further manipulation of the distal portion 20 and the energy delivery element(s) 24 within the respective renal artery RA establishes apposition and alignment between the energy delivery element 24 and tissue along an interior wall of the respective renal artery RA. For example, as shown in Figure 4, the mesh structure 22 is expanded within the renal artery RA such that the energy delivery element 24 is in contact with a renal artery wall 55. In some embodiments, manipulation of the distal portion 20 will also facilitate contact between the energy delivery element 24 and the wall 55 of the renal artery. The alignment may also include alignment of geometrical aspects of the energy delivery element 24 with the renal artery wall 55. For example, for embodiments in which the energy delivery element 24 has a cylindrical shape with rounded ends, alignment may include alignment of the longitudinal surface in contact with the artery wall 55. In another example, an embodiment may comprise an energy delivery element 24 with a structured shape or inactive surface, and alignment may include aligning the energy delivery element 24 such that the structured shape or inactive surface is not in contact with the artery wall 55.

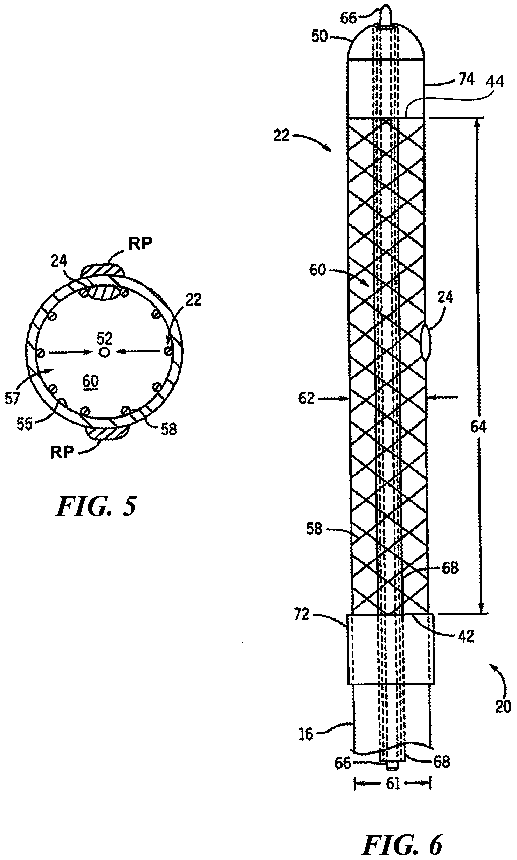

Figure 5 is a transverse cross-sectional view along line 5-5 of Figure 4 of the mesh structure 22 in the deployed or expanded configuration and in contact with the renal artery wall 55. One feature of the mesh structure 22 is that contact with the renal artery wall 55 is discontinuous. In particular, the mesh structure 22 includes fibers 58 or other solid structural elements separated by interstitial spaces 57. The mesh structure 22 expands and creates an interior space 60 that is accessible to blood flow via the series of interstitial spaces 57 in the mesh structure 22. Another feature of the mesh structure 22 is that, in particular embodiments, once inserted in a patient, expansion of the mesh structure is limited to the diameter 52 of the renal artery lumen. That is, the mesh structure 22 may, in certain embodiments, be more conformable than the renal artery such that the mesh structure 22 expands to span the lumen diameter 52 but does not apply sufficient radial force to the renal artery wall 55 to over-distend or injure the renal artery. Accordingly, an expanded diameter of the mesh structure 22 may, in some embodiments, be approximately equal to the lumen diameter 52 of the renal artery. Alternatively, as a renal artery lumen diameter 52 may vary from patient to patient, the mesh structure 22 may be configured to be deployed to a range of vessel lumen diameters. For example, a mesh structure 22 may be configured to expand, unconstrained, in diameter from a collapsed state to a fully deployed state with a diameter of about 10 mm and may be deployed within a renal artery until the mesh structure 22 and/or the energy delivery element 24 is/are in contact with the artery wall 55.

The mesh structure 22 may also be characterized by its diameter in the collapsed or delivery configuration, e.g., a smallest diameter. Figure 6, for example, is a side view of the distal end region 20 of the treatment device 12 in the collapsed configuration. A collapsed diameter 62 of the mesh structure 22 may be approximately equal to a diameter 61 of the elongated shaft 16. As noted above, for example, the sizing and dimension of the treatment device 12 may be configured to allow insertion with or without a guide catheter into a patient via an opening in the femoral, brachial, or radial arteries.

For practical purposes, the maximum outer dimension (e.g., diameter) of any section of the elongated shaft 16, including the energy delivery element 24 it carries, is dictated by the inner diameter of the guide catheter through which the elongated shaft 16 is passed. In one particular embodiment, for example, an 8 French guide catheter (having, for example, an inner diameter of approximately 0.091 inch, 2.31 mm) may be, from a clinical perspective, the largest guide catheter used to access the renal artery. Allowing for a reasonable clearance tolerance between the energy delivery element 24 and the guide catheter, the maximum outer dimension of the elongated shaft 16 may be expressed as being less than or equal to approximately 0.085 inch (2.16 mm). In such an embodiment, the mesh structure 22 (in a collapsed configuration and including the energy delivery element 24) may have a collapsed diameter 62 that is less than or equal to approximately 0.085 inch (2.16 mm). However, use of a smaller 5 French guide catheter may require smaller outer diameters along the elongated shaft 16. For example, a mesh structure 22 that is to be routed within a 5 French guide catheter would have an outer dimension of no greater than 0.053 inch (1.35 mm). In another example, the mesh structure 22 and energy delivery element 24 that are to be routed within a 6 French guide catheter would have an outer dimension of no greater than 0.070 inch (1.78 mm). In still further examples, other suitable guide catheters may be used, and outer dimension and/or arrangement of the shaft 16 can vary accordingly.

The mesh structure 22 may also be characterized by its length 64 in the collapsed configuration. In particular embodiments, it is envisioned that the length 64 may be measured from the proximal end 42 of the mesh structure 22 (e.g., at an interface of the proximal end 42 and any coupling 72 to the elongated shaft 16) to the distal end 44 of the mesh structure 22. Further, the length 64 in the collapsed configuration may generally be suitable for insertion into the renal artery. That is, the length may be approximately equal to or less than a renal artery length or a main renal artery (i.e. a section of a renal artery proximal to a bifurcation). As this dimension may vary from patient to patient, it is envisioned that in some embodiments the mesh structure 22 may be fabricated in different sizes (e.g., with different lengths 64 and/or diameters 62) that may be appropriate for different patients.

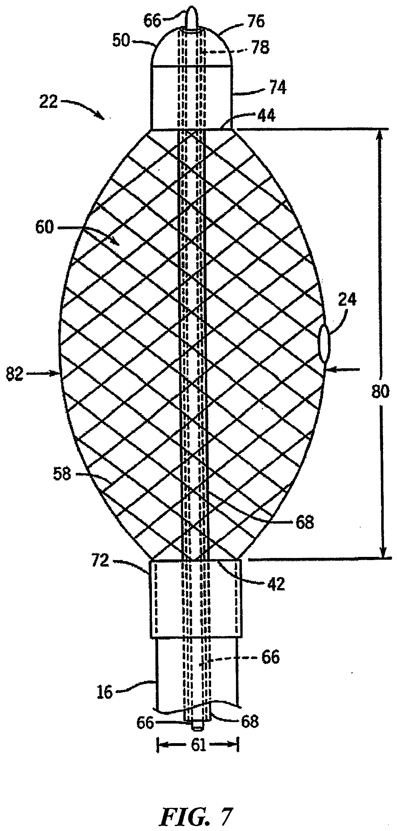

In one embodiment, the distal end 44 of the mesh structure 22 may be coupled to an end piece 74 (e.g., a collar, shaft, or cap) having a rounded distal portion 50 to facilitate atraumatic insertion of the treatment device 12 into a renal artery. In addition, the elongated shaft 16, the coupling 72, the mesh structure 22, and the end piece 74 may include passages sized and shaped to accommodate a control wire 68 that is fixed to the distal end 44 of the mesh structure or the end piece 74 and passes through the elongated shaft 16 to the proximal portion 18 of the elongated shaft 16. The control wire 68 facilitates the expansion and/or contraction of the mesh structure 22 when it is pulled or pushed to shorten or lengthen the mesh structure 22. For example, pulling (i.e., an increase in tension) the control wire 68 proximally relative to the shaft 16 may trigger expansion of the mesh structure 22 by drawing end piece 74 closer to coupling 72. Conversely, pushing (i.e., an increase in compression) the control wire 68 distally relative to shaft 16 may lengthen the mesh structure 22 to a compressed configuration by axially spreading apart end piece 74 and coupling 72. It will be understood that either the shaft 16 or the control wire 68 may be held in fixed position with respect to the patient while the other element is translated to create the relative movements described above. In some embodiments the mesh structure 22 has elastic or super-elastic shape memory properties such that when force is removed the mesh structure elastically returns to a relaxed state. Force may be applied by the control wire 68 to deform the mesh structure 22 into one state and when force is removed the mesh structure 22 returns to its relaxed state. For example, a relaxed state of the mesh structure 22 may be an expanded configuration as shown in Figure 7 and the control wire 68 may be pushed to lengthen the mesh structure 22 and reduce its diameter placing it in a collapsed configuration as shown in Figure 6. Alternatively, a relaxed state of the mesh structure may be a collapsed or compressed configuration and the control wire 68 may be pulled (tension applied) to shorten the mesh structure 22 and increase its diameter placing it in an expanded configuration.

In some embodiments the control wire 68 may be a solid or stranded wire or cable made from a metal or polymer. In other embodiments (such as the example shown in Figures 6 and 7) the control wire may be a hollow tube that can be passed over a guide wire 66 to facilitate insertion through an intravascular path to a renal artery.

The proximal end 42 of the mesh structure 22 may be coupled to the elongated shaft 16 via a coupling piece 72. Coupling piece 72, for example, may be an integral end of the elongated shaft 16 (e.g., may not be a separate piece) or may be a separate piece that is associated with the distal region 20 of the elongated shaft 16. The coupling piece 72 may be formed from the same type of material as the elongated shaft 16, or may be formed from a different material. In one embodiment, the coupling piece 72 may be formed from a collar, such as a radiopaque band, that surrounds and secures the mesh structure 22 to an exterior surface of the elongated shaft 16. In other embodiments, however, the coupling piece 72 may have a different arrangement and/or include different features.

Figure 7 is a side view of the mesh structure of Figure 6 in an expanded configuration. Referring to Figures 6 and 7 together, when not inserted into a patient, the mesh structure 22 is capable of expanding to a maximum diameter 82 that is larger than the collapsed diameter 62. Further, the mesh structure 22 may be sized so that the maximum diameter 82 is larger than the lumen diameter 52 of the renal artery. In some embodiments, for example, when inserted into a patient, the mesh structure 22 expands radially to span the renal artery lumen. In such an example, the largest transverse dimension of the mesh structure 22 is approximately or slightly less than (e.g., in embodiments in which the energy delivery element 24 fills some of the space) the diameter 52 of the renal artery lumen. A slight amount of vessel distension may be caused without undue injury and a mesh structure 22 may expand such that its largest transverse dimension is slightly more than the natural lumen diameter 52 of the renal artery, or such that an energy deliver element is slightly pressed into the wall of the renal artery. A mesh structure that causes slight and non-injurious distension of an artery wall may advantageously provide stable contact force between the energy delivery element 24 and the artery wall and/or hold the energy delivery element in place even as the artery moves with respiratory motion and pulsing blood flow. In further embodiments, the lumen diameter 52 can restrict the expansion of the mesh structure 22 and provide a limit to the maximum diameter 82. This restriction can cause the mesh structure 22 to form more of a cylindrical tapered shape than the prolate spheroid shape illustrated in Figure 7. Because this lumen diameter 52 varies from patient to patient, the mesh structure 22 may be capable of assuming a range of diameters between the compressed diameter 62 and the maximum diameter 82.

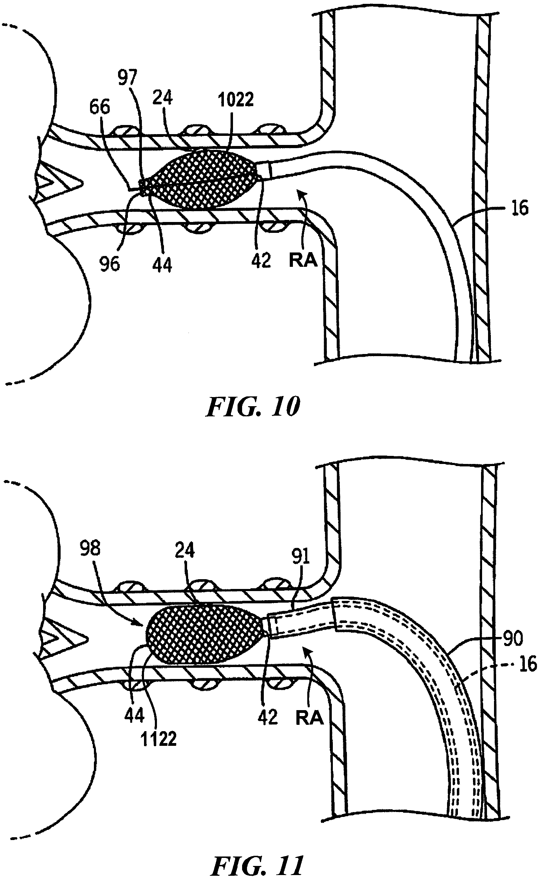

The mesh structure 22 in the expanded configuration may be characterized by its length 80 along the axis of the elongated shaft 16. In the depicted embodiment, only the proximal end 42 of the mesh structure 22 is coupled to the elongated shaft. As the mesh structure 22 expands, its diameter increases and its length decreases. That is, when the mesh structure expands, the distal end 44 moves axially towards the proximal end 42. Accordingly, the expanded length 80 can be less than the unexpanded, or collapsed, length 64 (see Figure 10). In certain embodiments, only one of the proximal end 42 or the distal end 44 of the mesh structure is fixedly coupled to the elongated shaft 16. In such a configuration, the distance between the proximal end 42 and the distal end 44 changes as the mesh structure 22 moves between the expanded and collapsed configurations. In further non-braided mesh embodiments, the length 80 does not decrease with the expansion of the mesh structure 22. As discussed further below with reference to Figure 8, for example, a delivery sheath can be used for deploying the mesh structure 22; in some embodiments the mesh structure 22 can self-expand and lengthen when the delivery sheath is retracted.

The dimensions of the mesh structure 22 are influenced by its physical characteristics and its configuration (e.g., expanded vs. unexpanded), which in turn may be selected with renal artery geometry in mind. For example, the axial length of the mesh structure 22 may be selected to be no longer than a patient's renal artery. Dimensions of the renal artery may be derived from textbooks of human anatomy, augmented with a clinician's knowledge of the site generally or as derived from prior analysis of the particular morphology of the targeted site. For example, the distance between the access site and the junction of the aorta and renal artery (e.g. the distance from a femoral access site to the renal artery is typically approximating about 40 cm to about 55 cm) is generally greater than the length of a renal artery between the aorta and the most distal treatment site along the length of the renal artery, which is typically less than about 7 cm. Accordingly, it is envisioned that the elongated shaft 16 is at least 40 cm and the mesh structure is less than about 7 cm in its unexpanded length 64, for example. A length in an unexpanded configuration of no more than about 4 cm may be suitable for use in a large population of patients and provide a long contact area in an expanded configuration and in some embodiments provide a long region for placement of multiple energy delivery elements; however, a shorter length (e.g. less than about 2 cm) in an unexpanded configuration may be used in patients with shorter renal arteries. The mesh structure 22 may also be designed to work with typical renal artery lumen diameters. For example, the lumen diameter 52 of the renal artery may vary between about 2 mm and about 10 mm. In a particular embodiment, the placement of the energy delivery element 24 on the mesh structure 22 may be selected with regard to an estimated location of the renal plexus relative to the renal artery.

As noted, the expanded configuration length 80 of the mesh structure 22 is less than the length 64 in the compressed configuration. In some embodiments, the length 80 may be less than about 50%, 55%, 60%, 65%, 70%, 75%, 80%, 85%, or 90% of the compressed length 64. Further, in some embodiments, the expanded configuration diameter 82 may be at least 1.2x, 1.25x, 1.5, 1.75x, 2x, 2.25x, 2.5x, 2.75x 3x, 3.25x, 3.5x, 3.75x, 4x, 4.25x, 4,5x, 4.75x, or 5x the compressed diameter 62.

The dimensions of the mesh structure 22 may be taken into account. That is, a typical renal artery may constrict, dilate or move relative to the aorta in response to blood flow changes or changes in a patient's breathing, etc. The mesh structure 22 may be selected to be used in conjunction with a particular renal artery lumen diameter 52, taking into account that this lumen diameter 52 may change (e.g., up to 20%) during the time that the mesh structure is in place. As such, the largest unconstrained diameter 82 of the mesh structure 22 may be sufficiently oversized relative to the renal artery to allow for additional expansion during use. In one embodiment, the unconstrained diameter 82 may be at least 1.2x, 1.5x, or 2x an estimated renal artery lumen diameter 52. In addition, as provided herein, stable contact with the renal artery is facilitated by the contact force of the mesh structure 22 against the renal artery wall 55. This contact force is influenced by the materials and construction of the mesh structure 22. The mesh structure 22 may be able to provide a substantially constant/stable contact force against the renal artery wall 55 within a particular range of diameters that the renal artery and the inserted mesh structure 22 jointly assume. In a particular embodiment, the contact force may be substantially stable over a range of diameters for example, between about 3 mm-5 mm, 5 mm-8 mm, or 6 mm-10 mm. In another embodiment the contact force may be suitable over a range of 2 mm-10 mm by controlling the amount of expansion or by suitable exertion of expansion force created by a self expanding mesh structure, for example by a mesh structure fabricated with super-elastic material such as nickel titanium alloy (nitinol) or composite nitinol with polymer coating for insulation.

**Table 1: Examples of approximate dimensions of mesh structures in expanded and unexpanded configurations.**

| Expanded configuration diameter (in patient) | Expanded configuration diameter (outside patient) | Compressed configuration diameter | Expanded configuration axial length (outside patient) | Compressed configuration axial length |
|---|---|---|---|---|
| 2 mm-8 mm | 3 mm-12 mm | <2 mm-<2.18 mm | <36 mm | <40 mm |
| 3 mm-8 mm | 5 mm-10 mm | <2.18 mm | <30 mm-<36 mm | <40 mm |
| 6 mm-8 mm | 8 mm-10 mm | <2.18 mm | <32 mm | <40 mm |
| 5 mm-8 mm | 6 mm-10 mm | <2.18 mm | <30 mm | <40 mm |
| 3 mm-5 mm | 5 mm-8 mm | <2.18 mm | <30 mm | <40 mm |

### A. Formation of the Mesh Structure

Referring to Figures 6 and 7 together, the mesh structure 22 includes structural elements, e.g., strands, wires, filaments or fibers 58, arranged to define interstices or interstitial spaces 57 there between. As provided above, the mesh structure 22 may be characterized by its axial length (e.g., an axial length in an expanded configuration or unexpanded configuration, either inside or outside the patient) or its diameter (e.g., a largest diameter in an expanded or unexpanded configuration, either inside or outside the patient). In addition, the mesh structure 22 may be characterized by the structural elements from which it is formed. Because the change in diameter and length between the expanded configuration and the collapsed configuration may involve realignment of strands 58 and changes in the geometry of the interstitial spaces 57, the makeup of the strands 58 and the geometry of the interstitial spaces 57 may at least in part define how much the diameter and length of the mesh structure 22 change as a result of configuration changes.

The fibers 58 may be formed from biocompatible metals, polymers, or composites. For example, suitable metals can include stainless steel, spring steel, cobalt chromium, gold, platinum, platinum-iridium, stainless steel, or combinations thereof. In embodiments in which the fibers 58 are composed solely of metal, the entire mesh structure 22 can comprise the electrode 24. For example, in one particular embodiment, the mesh structure 22 may be composed of nitinol with gold plating to enhance radiopacity and/or conductivity. Suitable polymer materials can include, for example, polyethylene terephthalate (PET), polyamide, polyimide, polyethylene block amide copolymer, polypropylene, or polyether ether ketone (PEEK) polymers. In still further embodiments, the mesh structure 22 may be a combination of electrically conductive and nonconductive materials.

In addition, in particular embodiments, the mesh structure 22 may be formed at least in part from radiopaque materials that are capable of being imaged fluoroscopically to allow a clinician to determine if the mesh structure 22 is appropriately placed and/or deployed in the renal artery. Radiopaque materials may include barium sulfate, bismuth trioxide, bismuth subcarbonate, powdered tungsten, powdered tantalum, or various formulations of certain metals, including gold and platinum, and these materials may be directly incorporated into the fibers 58 or may form a partial or complete coating of the mesh structure 22.

Generally, the mesh structure 22 may be designed to apply a desired outward radial force to a renal artery wall when inserted and expanded to contact the inner surface of the renal artery wall 55. The radial force may be selected to avoid injury from stretching or distending the renal artery when the mesh structure 22 is expanded against an artery wall within the patient. Radial forces that may avoid injuring the renal artery yet provide adequate stabilization force may be determined by calculating the radial force exerted on an artery wall by typical blood pressure. For example, a suitable radial force may be less than about 300 mN/mm (e.g. less than 200 mN/mm). In other embodiments, however, the radial force can vary. Factors that may influence the applied radial force include the geometry and the stiffness of the mesh structure 22. In one particular embodiment, for example, the fibers 58 are about 0.005-0.009 inch (0.330-1.23 mm) in diameter. Depending on the composition of the fibers 58, the fiber diameters and quantity of fibers may be selected to facilitate a desired conformability and/or radial force against the renal artery when expanded. For example, fibers 58 formed from stiffer materials (e.g. metals) may be thinner relative to fibers 58 formed highly flexible polymers to achieve similar flexibilities and radial force profiles. The outward pressure of the mesh structure 22 may be assessed *in vivo* by an associated pressure transducer.

Mesh structures 22 with open structures (e.g., low material-per-square-inch ratios) may have less radial stiffness and strength than more closed structures (or high material density structures). The fiber thickness also affects outward pressure, radial strength and stiffness. A thicker fiber 58 provides greater radial strength and stiffness compared with a relatively thinner fiber 58 of the same material. However, a stiffer fiber material may compensate for a generally open braid structure. In addition, certain secondary processes, including heat treating and annealing, may harden or soften the fiber material to affect strength and stiffness. In particular, for shape-memory alloys such as nitinol, these secondary processes may be varied to give the same starting material different final properties. For example, the elastic range or softness may be increased to impart improved flexibility. The secondary processing of shape memory alloys influences the transition temperature, i.e., the temperature at which the structure exhibits a desired radial strength and stiffness. This temperature may be set at normal body temperature (e.g., 37°C).

The mesh structure 22 may be laser cut, braided, knit, or woven to form a conformable structure (e.g., a tubular, barrel-shaped, parachute-shaped, or spherical structure) through which fluids may pass. In embodiments in which the mesh structure 22 is braided, the characteristics of the structure 22 may be influenced by the number of fibers. In a particular embodiment, the mesh structure 22 may have 8-96 fibers. It should be understood that a fiber may be formed from a single filament (monofilament) or by a plurality of filaments twisted or otherwise grouped together to form a multifilar fiber. In addition, the mesh structure 22 may be characterized by its braid pitch, which in embodiments may be between 10-90 picks (i.e., windings) per inch (3.9-35.5 picks per cm) or by its braid angle, defined as the angle between two intersecting braid strands and encompassing a longitudinal axis of the mesh structure 22. The braid angle of the mesh structure 22 in its expanded configuration may be in the range of 20° to 160° (e.g. about 100°). Further, the mesh structure 22 may be helically braided (e.g., clockwise and counterclockwise helices) into a generally ovoid, tubular, barrel, or other shaped structure. Additionally, the type of braiding process used to form the mesh structure 22 may influence its compressibility. For example, filaments braided in a pattern known as "two over and two under" will have greater bending stiffness than a simpler "one over and one under" pattern.

It should be understood that the mesh structure 22 may be generally symmetrical and coaxial with respect to the elongated shaft 16. However, it is also contemplated that the mesh structure 22 may be preformed to conform to any irregularities in the renal artery, which may be assessed by imaging or other techniques. For example, particular sizes and types of mesh structures may be used in conjunction with a patient's particular anatomic features.

### B. Additional Embodiments of Treatment Devices Having Mesh Structures

Figures 8-12B illustrate additional embodiments of treatment devices including expandable mesh structures configured in accordance with embodiments of the technology. The embodiments described below may have many of the same or similar features as the treatment devices described above, and may be used with the system 10 or other suitable systems for renal neuromodulation. Further, the embodiments provided herein include features that may be combined with one another and with the features of other disclosed embodiments. In an effort to provide a concise description of these embodiments, not all features of an actual implementation are described herein.

Figure 8, for example, illustrates a treatment device 812 used in conjunction with a delivery sheath 91 surrounds a mesh structure 822 and the elongated shaft 16. As noted above, in certain embodiments it may be advantageous to use a guide catheter 90 of a particular size to facilitate insertion of the treatment device 812 through the femoral artery. A delivery sheath 91 may be used in conjunction with a guide catheter 90 to gain access to a renal artery and deliver a radially constrained expandable mesh structure 822. Alternatively, a delivery sheath 91 may be used in conjunction with a guide wire (e.g., the guide wire 66 illustrated in Figure 7). When used in conjunction with a guide catheter 90, a working length of the elongated shaft 16 may be about 40 cm to about 125 cm. If, for example, a 55 cm length guide catheter is used, then this working length of the shaft 16 may be about 70 cm to about 80 cm. If a 90 cm length guide catheter is used, then this shaft working length may be about 105 cm to about 115 cm.

In the depicted embodiment, the mesh structure 822 may be held in the radially compressed configuration by the delivery sheath 91. Removal of the delivery sheath 91 allows the mesh structure 822 to expand radially so that the energy delivery element 24 is in proper apposition with the inner wall of the renal artery for energy delivery. The expansion may be passive (e.g., the mesh structure may be self-expanding or may expand as the mesh structure is filled with blood) or active (e.g., the expansion is facilitated by an interior balloon or fluid injection into the interior space of the mesh structure 822, or by a tension or control wire pulling on the distal end and/or pushing on the proximal end of a mesh structure 822 reducing its length to expand its diameter) Regardless of the type of expansion, the mesh structure 822 may by coupled to a control wire (e.g., the control wire 68 illustrated in Figure 6) that may aid in compressing the mesh structure 822 prior to removal or repositioning of the treatment device 812. In particular embodiments, depending on the placement and number of energy delivery elements 24, the mesh structure 822 may be selectively repositioned within the renal artery to provide a number of locations for energy delivery. The mesh structure 822 is expected to provide stable contact of the energy delivery element 24 with the inner wall of the renal artery without occluding the blood flow within the artery. To that end, the mesh structure 822 may be shaped to include open areas to help maintain normal blood flow.

Figure 9 illustrates a mesh structure 922 having an opening 994 at a distal end 44. A proximal end 42 of the mesh structure 922 is coupled to the elongated shaft 16, while the distal end 44 terminates in a terminal circumferential section 92 that is approximately the same diameter as the renal artery lumen diameter 52. The terminal circumferential section 92 may be bound or otherwise woven to eliminate any protruding fiber ends. In addition, a terminal circumferential section 92 may be relatively stiffer to prevent folding the free distal end 44. The substantially cylindrical mesh configuration 922 may provide more surface area for contacting the renal artery inner wall as compared to a more ball-shaped mesh structure, thus providing more potential surface area for mounting energy delivery elements 24.

Alternatively, as shown in Figure 10, a mesh structure 1022 may be shaped to include a relatively smaller distal opening 96 sized for receiving the guide wire 66 and limiting lateral deflection of the guide wire 66. The mesh in a neck 97 of the opening 96 may be relatively stiffer to help hold the guide wire 66 in place similar to the end piece 74 illustrated in Figure 7. In some embodiments, this arrangement can help maintain a desired alignment between the mesh structure 1022 and the vessel. (e.g., the mesh structure 1022 remains approximately centered in the artery with wall apposition forces substantially evenly displaced over its surface). Figure 11 shows an embodiment in which a mesh structure 1122 includes a closed end 98.

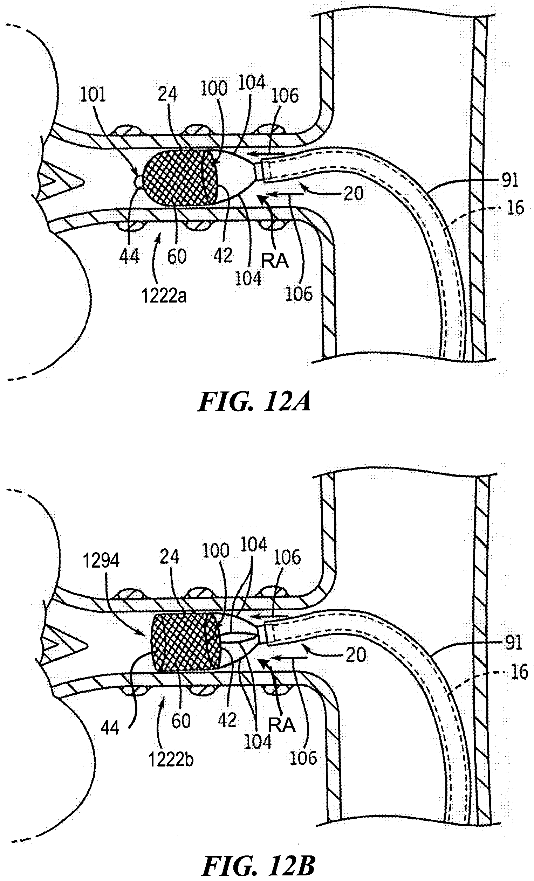

As noted previously, expansion of the mesh structure may be facilitated by blood flood within the renal artery. Figure 12A, for example, illustrates an embodiment in which the distal end 44 of a mesh structure 1222a is closed and the open proximal end 42 is axially spaced apart from the distal end of the elongated shaft 16. The blood flow depicted by arrows 106 may flow into the opening 100 to open the umbrella-like or parachute-like mesh structure 1222a with hydrodynamic force, pushing the energy delivery element 24 against the renal artery wall. The proximal end 42 of the mesh structure 1222a is coupled to the elongated shaft 16 via tethers or wires 104. In some embodiments, for example, the wires 104 may facilitate retraction of the mesh structure 1222a into a delivery sheath for removal or repositioning. In another embodiment, the distal end 44 of the mesh structure 1222a, rather than the proximal end 42, is coupled to the elongated shaft 16 via tethers that, when pulled, expand the mesh structure 22. In still further embodiments, the mesh structure 1222a is wired or tethered to the shaft 16 at both the proximal 42 and distal ends 44 and the mesh structure 1222a may be expanded or collapsed by moving the tether attachment points on the proximal end 42 and distal end 44 relative to each other (e.g., the distal end 44 could be pulled proximally to expand and pushed distally to collapse; the proximal end 42 could be pushed distally to expand or pulled proximally to collapse).

Referring next to Figure 12B, a mesh structure 1222b configured in accordance with another embodiment of the technology may have an opening 1294 at the distal end 44 in addition to the opening 100 at the proximal end 42 to allow maximum blood flow therethrough. In further embodiments, both ends of the mesh structure 1222b can be closed and/or tethered to the elongated shaft 16. In this embodiment the mesh may be self expanding. For example, the mesh structure 1222b may be made from an elastic or super elastic material such as nitinol or spring temper stainless steel and have an unrestrained configuration comprising an expanded diameter. The self expanding mesh structure 1222b may be deployed by retracting a delivery sheath 91 to remove constraining forces. The self expanding mesh structure 1222b may be compressed by retraction into the delivery sheath facilitated by pulling the wires 104. Wires 104 may further be beneficial by providing a flexible connection between the mesh structure 1222b and the distal end of the elongated shaft 16 such that if the distal end of the elongated shaft is eccentrically positioned within the artery, then the mesh structure 1222b remains centered in the artery with wall apposition forces substantially evenly displaced over its surface.

### C. Size and Configuration of the Energy Delivery Element(s)

It will be appreciated that the embodiments provided herein may be used in conjunction with one or more energy delivery elements 24. Referring to Figures 1-7 together, for example, the energy delivery element 24 associated with the mesh structure 22 may be a separate element or may be an integral part of the mesh structure 22. In some patients, it may be desirable to use the energy delivery element(s) 24 to create either a single lesion or a pattern of multiple focal lesions that are spaced apart circumferentially and/or axially along the longitudinal axis of the renal artery. A single focal lesion with desired longitudinal and/or circumferential dimensions, one or more full circumferential lesions, multiple circumferentially spaced focal lesions at a common longitudinal position, spiral-shaped lesions, interrupted spiral lesions, generally linear lesions, and/or multiple longitudinally spaced focal lesions along a line parallel to the axis of the renal artery alternatively or additionally may be created. In still further embodiments, the energy delivery element(s) 24 may be used to create lesions having a variety of other geometric shapes or patterns.

Depending on the size, shape, and number of the energy delivery elements 24, the lesions created may be circumferentially spaced around the renal artery, either in a single transverse plane or the lesions may also be spaced apart longitudinally. In particular embodiments, it is desirable for each lesion to cover at least 10% of the vessel circumference to increase the probability of affecting the renal plexus. It is also desirable that each lesion be sufficiently deep to penetrate into and beyond the adventitia to thereby affect the renal plexus. However, lesions that are too deep run the risk of interfering with non-target tissue and tissue structures (e.g., the renal vein) so a controlled depth of energy treatment is also desirable.

In certain embodiments, the energy delivery element 24 may be circumferentially repositioned relative to the renal artery during treatment. This angular repositioning may be achieved, for example, by compressing the mesh structure and rotating the elongated shaft 16 of treatment device 12 via handle assembly 34. In addition to the angular or circumferential repositioning of the energy delivery element 24, the energy delivery element 24 optionally may also be repositioned along the lengthwise or longitudinal dimension of the renal artery. This longitudinal repositioning may be achieved, for example, by translating the elongated shaft 16 of the treatment device 12 via the handle assembly 34, and may occur before, after, or concurrently with angular repositioning of the energy delivery element 24. Repositioning the energy delivery element 24 in both the longitudinal and angular dimensions places the energy delivery element in contact with the interior wall of the renal artery at a second treatment site for treating the renal plexus. Energy then may be delivered via the energy delivery element 24 to form a second focal lesion at this second treatment site. For embodiments in which multiple energy delivery elements 24 are associated with the mesh structure, the initial treatment may result in two or more lesions, and repositioning may allow additional lesions to be created. One or more additional focal lesions optionally may be formed via additional repositioning of the mesh structure 22.

In certain embodiments, the lesions created via repositioning of the mesh structure 22 are circumferentially and longitudinally offset from the initial lesion(s) about the angular and lengthwise dimensions of the renal artery, respectively. The composite lesion pattern created along the renal artery by the initial energy application and all subsequent energy applications after any repositioning of the energy delivery element(s) 24 may effectively result in a discontinuous lesion (i.e., it is formed from multiple, longitudinally and angularly spaced treatment sites). To achieve denervation of the kidney, it may be desirable for the composite lesion pattern, as viewed from a proximal or distal end of the vessel, to extend at least approximately all the way around the circumference of the renal artery. In other words, each formed lesion covers an arc of the circumference, and each of the lesions, as viewed from an end of the vessel, abut or overlap adjacent lesions to create a virtually circumferential lesion. The formed lesions defining an actual circumferential lesion lie in a single plane perpendicular to a longitudinal axis of the renal artery. A virtually circumferential lesion is defined by multiple lesions that may not all lie in a single perpendicular plane, although more than one lesion of the pattern can be so formed. At least one of the formed lesions comprising the virtually circumferential lesion is axially spaced apart from other lesions. In a non-limiting example, a virtually circumferential lesion can comprise six lesions created in a single helical pattern along the renal artery such that each lesion spans an arc extending along at least one sixth of the vessel circumference such that the resulting pattern of lesions completely encompasses the vessel circumference, when viewed from an end of the vessel. In other examples, however, a virtually circumferential lesion can comprise a different number of lesions

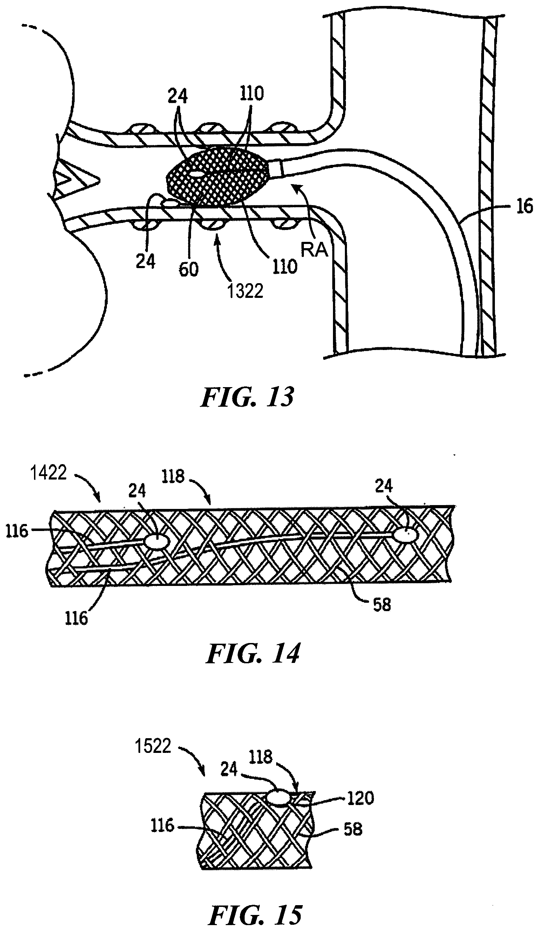

In one example, as shown in Figure 13, a mesh structure 1322 (e.g., an open or closed-ended structure) may function as an expandable member to radially push multiple energy delivery elements 24 coupled to leads 110 against the inner wall of the renal artery. In the depicted embodiment, the leads 110 may be separate from the mesh structure 1322, or may be loosely coupled or integrated into to the mesh structure 1322 to prevent twisting or kinking of the leads 110. In particular embodiments, to facilitate the stable contact of the energy delivery element 24 in the renal artery, the energy delivery element 24 may be coupled to a mesh structure 1422 by weaving a lead 116 into the fibers 58 of the mesh or threading leads 116 through interstices in the mesh, as shown in Figure 14. The energy delivery elements 24 are positioned on an exterior surface 118 or in spaces of the mesh structure 1422. The positioning of the energy delivery elements 24 on the exterior surface 118 may be associated with a desired lesion pattern. Alternatively, as shown in Figure 15, the energy delivery element 24 may be directly coupled to the fibers 58 of a mesh structure 1522. The energy delivery element 24 is coupled to one or more fibers 58, for example via adhesion or threading a fiber 58 through an internal bore 120.

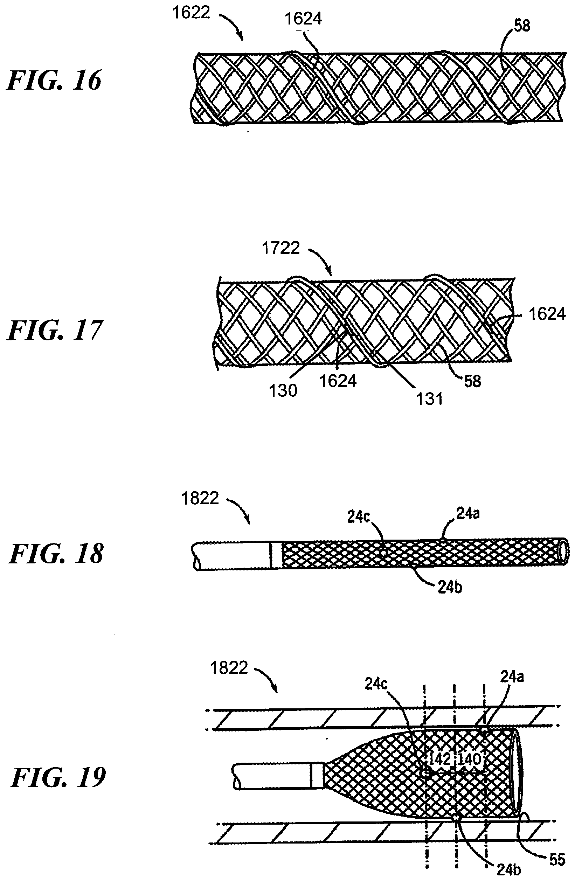

In an alternative embodiment, the energy delivery element 24 may be in the form of an electrically conductive wire or cable, e.g., a ribbon electrode. As shown in Figure 16, the ribbon electrode 1624 may be wound about a mesh structure 1622 or may be woven into or otherwise coupled to the mesh structure 1622. The ribbon electrode 1624 may provide increased surface area for delivering energy. For example, the ribbon electrode 1624 may form a helical lesion in a single energy application. Accordingly, the mesh structure 1622 may be capable of providing sufficient renal denervation with a single energy application at a single location.. The ribbon electrode 1624 may be wound in any manner about the mesh structure 1622, depending on the desired lesion to be formed. For example, the ribbon electrode 1624 may form a loose-pitch or tight-pitch helix. In addition, the winding may be tight against the mesh structure 1622, so that the ribbon electrode 1624 generally follows the contours of the mesh structure 1622. In other embodiments, slack portions of the ribbon electrode 1624 may be pulled into the interior space to allow the forming of discontinuous lesions. Further, in such an arrangement, regions of the ribbon electrode 1624 that do not contact the renal artery wall may contribute to cooling of the energy delivery element 24, as provided herein. Alternatively, as shown in Figure 17, only portions 130 of the ribbon electrode 1624 may be electrically conductive with the vessel tissue. That is, the ribbon electrode 1624 can include insulated portions 131 and uninsulated portions 130 in which the insulation is removed. The positioning and number of stripped portions 130 forming the energy delivery elements 1624 may be selected according to a desired lesion pattern to be formed.

As noted, one or more energy delivery elements 24 may be associated with the mesh structure 22 for forming a particular lesion pattern. As shown in Figure 18, energy delivery elements 24a, 24b, and 24c may be distributed on the mesh structure 1822. The axial distances (e.g., distances 140 and 142 in Figure 19) between axially adjacent energy delivery elements 24 may be selected so that the edges of the lesions formed by each individual energy delivery elements 24 on the renal artery wall 55 are either overlapping or nonoverlapping. One or both of the axial distances 140 or 142 may be about 2 mm to about 1 cm. In a particular embodiment, the axial distances 140 or 142 may be in the range of about 2 mm to about 5 mm. In another representative embodiment, the axially adjacent energy delivery elements 24 may be spaced apart about 30 mm. In another representative embodiment, the axially adjacent energy delivery elements 24 are spaced apart about 11 mm. In still another representative embodiment, the axially adjacent energy delivery elements 24 are spaced apart about 17.5 mm. Further, the axial distance 140 may be less than, about equal to, or greater than the axial distance 142.

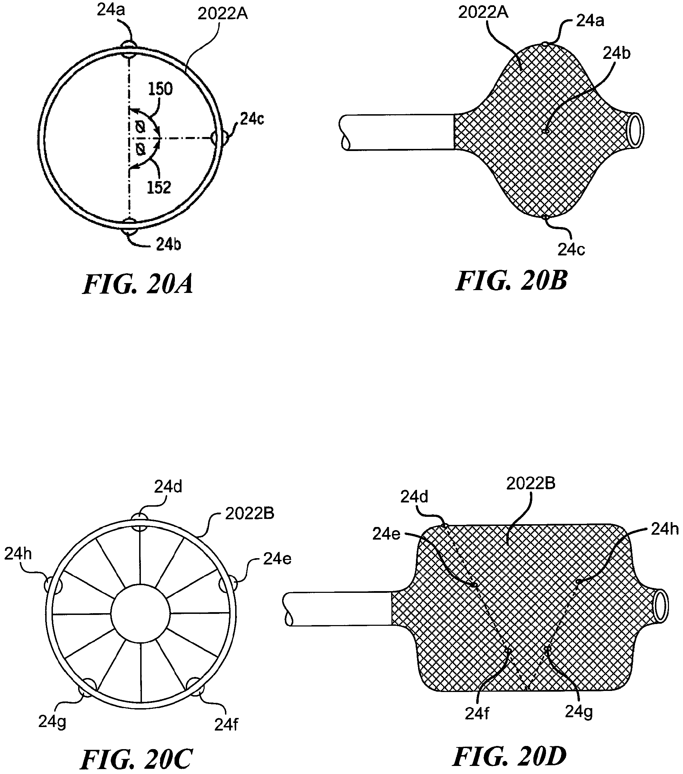

In some embodiments, the energy delivery elements 24 are both longitudinally and circumferentially offset from one another. For example, Figure 20A is an end view of a mesh structure 2022A in which the energy delivery elements 24a-24c are affixed to the mesh pattern in such an arrangement that element 24c is circumferentially offset from energy delivery element 24a by angle 150 and circumferentially offset from energy delivery element 24b by angle 152. Figure 20B is a side view of the mesh structure 2022A illustrating the energy delivery elements 24a, 24b, and 24c in a generally circumferentially aligned arrangement. Figure 20C is an end view of a generally cylindrical mesh structure 2022B having energy delivery elements affixed to the mesh structure 2022B in a helical pattern such that elements 24d-24h are circumferentially and axially offset from one another. Figure 20D is a side view of the mesh structure 2022B. The circumferential offset arcs, or corresponding radial angles may be selected so that when energy is applied to the renal artery via energy delivery elements 24d-24h, a roughly helical lesion pattern is formed therein. Depending on the number and positioning of the energy delivery elements 24 selectively mounted on mesh structure 2022B, a helical lesion pattern with any desired number of turns may be formed with treatment device 12 using only a single energy application. In other embodiments, the energy delivery elements 24 may have a variety of different arrangements relative to each other (e.g., linear, interrupted helix, continuous helix).

As discussed previously, the energy delivery element 24 is sized and configured to contact an internal wall of the renal artery during operation. For example, referring back to Figures 1 and 2, the energy delivery element 24 may take the form of an electrode sized and configured to apply an electrical field of RF energy from the energy generator 26 (Figure 1) to a vessel wall. The energy delivery element 24 may be operated in a monopolar or unipolar mode. In this arrangement, a return path for the applied RF electric field is established, e.g., by an external dispersive electrode (e.g., the external dispersive electrode 38 in Figures 1 and 2), also called an indifferent electrode or neutral electrode. The monopolar application of RF electric field energy serves to ohmically or resistively heat tissue in the vicinity of the electrode 24. The application of the RF electrical field thermally injures tissue. The treatment objective is to thermally induce neuromodulation (e.g., necrosis, thermal alteration or ablation) in the targeted neural fibers. The thermal injury forms a lesion in the vessel wall. Alternatively, a RF electrical field may be delivered with an oscillating intensity that does not thermally injure the tissue whereby neuromodulation in the targeted nerves is accomplished by electrical modification of the nerve signals.

The active surface area of the energy delivery element 24 is defined as the energy transmitting area of the element 24 that may be placed in intimate contact against tissue. Too much contact between the energy delivery element and the vessel wall may create unduly high temperatures at or around the interface between the tissue and the energy delivery element, thereby creating excessive heat generation at this interface. This excessive heat may create a lesion that is circumferentially too large. This may also lead to undesirable thermal application to the vessel wall. In some instances, too much contact can also lead to small, shallow lesions. Too little contact between the energy delivery element and the vessel wall may result in superficial heating of the vessel wall, thereby creating a lesion that is too small (e.g., < 10% of vessel circumference) and/or too shallow.

The active surface area (ASA) of contact between the energy delivery element 24 and the inner vessel wall (e.g., the renal artery wall 55 of Figures 3-5) has great bearing on the efficiency and control of the generation of a thermal energy field across the vessel wall to thermally affect targeted neural fibers in the renal plexus RP. While the active surface area (ASA) of the energy delivery element is important to creating lesions of desirable size and depth, the ratio between the active surface area (ASA) and total surface area (TSA) of the energy delivery element 24 and electrode 46 is also important. The ASA to TSA ratio influences lesion formation in two ways: (1) the degree of resistive heating via the electric field, and (2) the effects of blood flow or other convective cooling elements such as injected or infused saline. For example, an RF electric field causes lesion formation via resistive heating of tissue exposed to the electric field. The higher the ASA to TSA ratio (i.e., the greater the contact between the electrode and tissue), the greater the resistive heating, e.g., the larger the lesion that is formed. As discussed in greater detail below, the flow of blood over the noncontacting portion of the electrode (TSA minus ASA) provides conductive and convective cooling of the electrode, thereby carrying excess thermal energy away from the interface between the vessel wall and electrode. If the ratio of ASA to TSA is too high (e.g., more than 50%), resistive heating of the tissue may be too aggressive and not enough excess thermal energy is being carried away, resulting in excessive heat generation and increased potential for stenotic injury, thrombus formation and undesirable lesion size. If the ratio of ASA to TSA is too low (e.g., 10%), then there is too little resistive heating of tissue, thereby resulting in superficial heating and smaller and shallower lesions. In a representative embodiment, the ASA of the energy delivery elements 24 contacting tissue may be expressed as
0.25 TSA ≤ ASA ≤ 0.50 TSA

An ASA to TSA ratio of over 50% may still be effective without excessive heat generation by compensating with a reduced power delivery algorithm and/or by using convective cooling of the electrode by exposing it to blood flow. As discussed further below, electrode cooling can be achieved by injecting or infusing cooling fluids such as saline (e.g., room temperature saline or chilled saline) over the electrode and into the blood stream.

Various size constraints for an electrode energy delivery element 24 may be imposed for clinical reasons by the maximum desired dimensions of the guide catheter, as well as by the size and anatomy of the renal artery lumen itself. Typically, the maximum outer diameter (or cross-sectional dimension for non-circular cross-section) of the energy delivery element 24 is the largest diameter encountered along the length of the elongated shaft 16 distal to the handle assembly 34. As previously discussed, for clinical reasons, the maximum outer diameter (or cross-sectional dimension) of the energy delivery element 24 is constrained by the maximum inner diameter of the guide catheter through which the elongated shaft 16 is to be passed through the intravascular path 14. For example, as provided above, assuming that an 8 French guide catheter (which has an inner diameter of approximately 0.091 inch or 2.31 mm) is, from a clinical perspective, the largest desired catheter to be used to access the renal artery, and allowing for a reasonable clearance tolerance between the energy delivery element 24 and the guide catheter, the maximum diameter of the electrode 46 is constrained to about 0.085 inch or 2.16 mm. In the event a 6 French guide catheter is used instead of an 8 French guide catheter, then the maximum diameter of the energy delivery element 24 is constrained to about 0.070 inch or 1.78 mm. In the event a 5 French guide catheter is used, then the maximum diameter of the energy delivery element 24 is constrained to about 0.053 inch or 1.35 mm.

In one embodiment, the energy delivery element 24 can take the form of a cylinder or a ball. Based upon these constraints and the aforementioned power delivery considerations, the energy delivery element 24 may have an outer diameter of from about 0.049 to about 0.051 inch (1.24 mm-1.30 mm). The energy delivery element 24 also may have a minimum outer diameter of about 0.020 inch or 0.51 mm to provide sufficient cooling and lesion size. In some embodiments, the energy delivery element 24 may have a length of about 1 mm to about 3 mm. In some embodiments in which the energy delivery element 24 is a resistive heating element, the energy delivery element 24 has a maximum outer diameter from about 0.049 to 0.051 inch (1.24 mm-1.30 mm) and a length of about 10 mm to 30 mm.

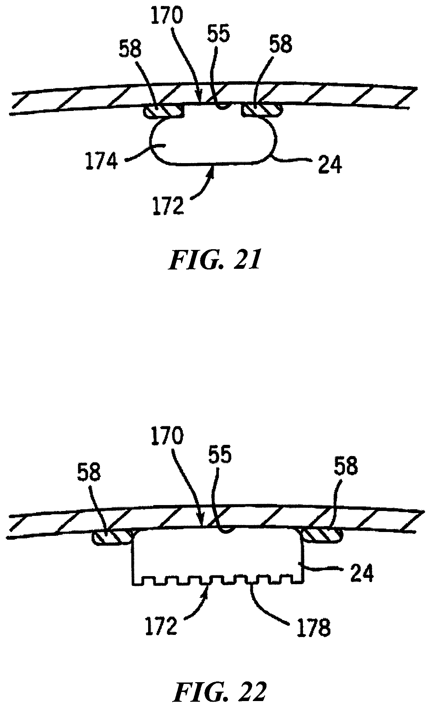

In other embodiments, cooling of the energy delivery element 24 may be facilitated by having an irregularly or asymmetrically shaped energy delivery element. For example, referring to Figures 21 and 22, an energy delivery element 24 positioned among fibers 58 of a mesh structure may have an active surface 170 configured to contact the renal artery wall 55 and an inactive surface 172. The inactive surface 172 is larger than the active surface 170 because of asymmetry in the energy delivery element 24. As shown in the illustrated embodiments, this may be because of a bulbous projection 174, or because of surface projections 178 (e.g., teeth) that form an irregular inactive surface 172. The inactive surface acts as a heat sink for the energy delivery element 24 because more surface area of the electrode energy delivery element 24 is exposed to the cooling blood flow. As such, in particular embodiments, the energy delivery element 24 may be formed in any shape in which the surface area of the inactive surface 172 is larger than the surface area of the active surface 170. In alternative embodiments, a ribbon electrode energy delivery element 24 may be formed so that an inactive surface area is larger than an active surface area. As noted, this may be accomplished by forming a certain amount of slack into the winding and pulling portions of the ribbon electrode into the interior space 60. Alternatively, the ribbon electrode may be formed to have asymmetrical regions.

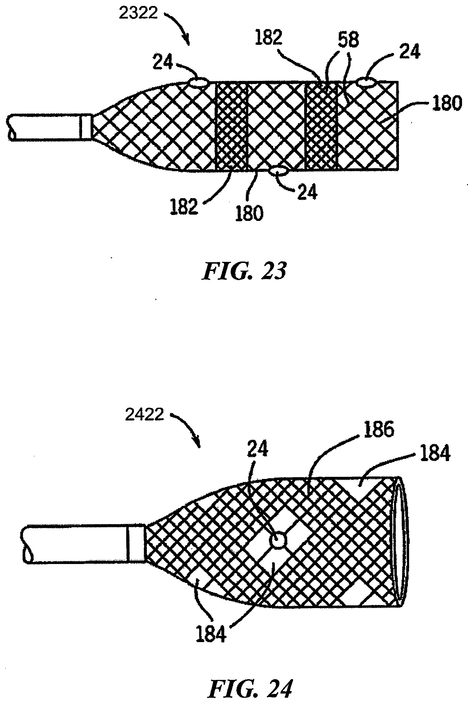

The mesh structure 22 may also be altered to facilitate blood-mediated cooling of the energy delivery element 24. Figure 23 illustrates an embodiment in which a mesh structure 2322 features axially arranged cylindrical sections of different mesh density (e.g., different braid pitch). Sections 180 that include an energy delivery element may alternate with sections 182 that do not include an energy delivery element 24. The sections 180 have a more open mesh relative to the sections 182 that do not include energy delivery elements 24. This arrangement may encourage blood flow in and around the more open mesh surrounding the energy delivery elements 24. In one embodiment, the alternating sections 180 and 182 may be braided as part of a single structure, with varying braid pitch as the mesh structure 2322 is formed.

Figure 24 illustrates an alternative embodiment including a mesh structure 2422 having regions 184 of relatively open mesh surrounding the energy delivery elements 24 and denser mesh 186 in the remaining portions of the mesh structure 2422. Such a mesh structure 2422 may be formed by varying braid pitch or by laser cutting the regions 184 and 186. Further, it should be understood that a single mesh structure 2422 may include 2, 3, or more regions each with a different mesh pitch, as appropriate.

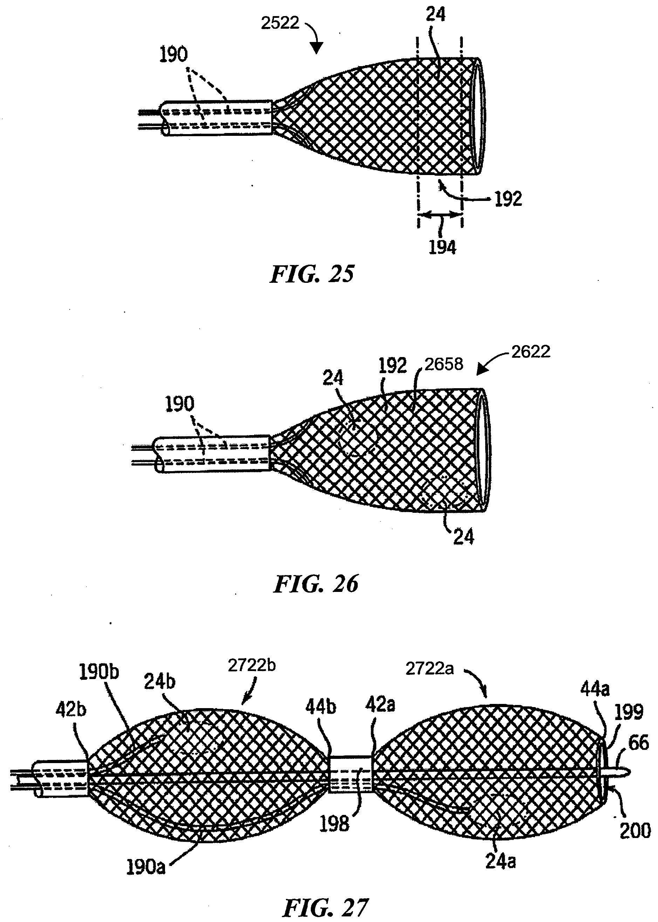

In certain embodiments, the mesh structure 22 may be formed of an electrically conductive material. Referring to Figure 25, for example, wire leads 190 may connect the energy generator 26 and conductive mesh structure 2522. The mesh structure 2522 forms a contact region 192 that contacts the renal artery inner wall and acts as the energy delivery element 24. Depending on the shape of the mesh structure 2522, the contact region 192 may form a band with a width 194 that corresponds to the portion of the mesh structure 2522 that expands to contact the renal artery wall. In this configuration, the mesh structure 2522 is capable of producing a circumferential lesion. The lesion may be wider for more tube or barrel-shaped mesh structures 2522 or narrower for more spherical mesh structures 2522. Accordingly, depending on the strength of power applied via the energy delivery element 24 and the particular situation of each patient, the mesh structure 2522 may be designed so that the contact region is as wide or narrow as desired. In certain embodiments, the contact band has a width 194 of at least 1 mm, 2 mm, 3 mm, 4 mm, 5 mm, or 10 mm.

In other embodiments, the electrically conductive mesh structure 22 is at least partially insulated. For example, the fibers 58 can be metal wires covered with an electrically insulating material and portions of the insulating material may be stripped away to expose one or more energy delivery elements 24. The energy delivery elements 24 may be any size, shape, or number, and may be positioned relative to one another as provided herein. For example, one or more circumferential bands may be created along the length of the mesh structure 22. The bands may be formed of a desired width by removing a desired amount of insulating material from the mesh structure 22. Alternatively, individual sectors or quadrants (on the external and/or internal portions of the mesh structure 22) or selected filaments may have their insulation removed. The insulation can be removed from the fibers 58 in a variety of ways to create the stripped portions that serve as conductive energy delivery elements 24. For example, the insulation may be scraped away or ablated, e.g., by a thermal radiation source such as a laser. Further, the energy delivery elements may be formed by masking selected portions of the mesh structure 22 that are intended to remain insulated after laser ablation (of the unmasked portions).

As shown in Figure 26, a mesh structure 2622 itself may be formed from an insulated electrically conductive material. In such embodiments, any individual energy delivery element 24 formed by selective removal of insulation to expose the electrically conductive material may be electrically connected to the other energy delivery elements 24 on the mesh structure 2622. Electrically conductive fibers 2658 of the mesh structure 2622 may be formed (e.g., braided) so that spaced apart locations on the mesh structure 24 are electrically connected to one another. In such an arrangement, in order to obtain electrically independent energy delivery elements 24 (i.e., that may be activated and operated separately) additional mesh structures 22 may be associated with the treatment device. For example, as shown in Figure 27, mesh structures 2722a and 2722b each include respective energy delivery elements 24a and 24b. A proximal end 42a of the mesh structure 2722a and a distal end 44b of the mesh structure 22b may be joined via a joining piece 198 that electrically insulates the two structures. Energy delivery element 24a is coupled to a wire lead(s) 190a, while energy delivery element 24b is coupled to a separate wire lead(s) 190(b). By independently supplying power to wire leads 190a or 190b, energy delivery elements 24a and 24b may be activated separately. As shown, the mesh structures 2722a and 2722b may be used in conjunction with guide wire 66. In such an embodiment, the joining piece 198 may include an internal passage sized to accommodate the guide wire and the distal end 44a of the mesh structure 2722a may also feature an opening 199 that permits passage of the guide wire 66.

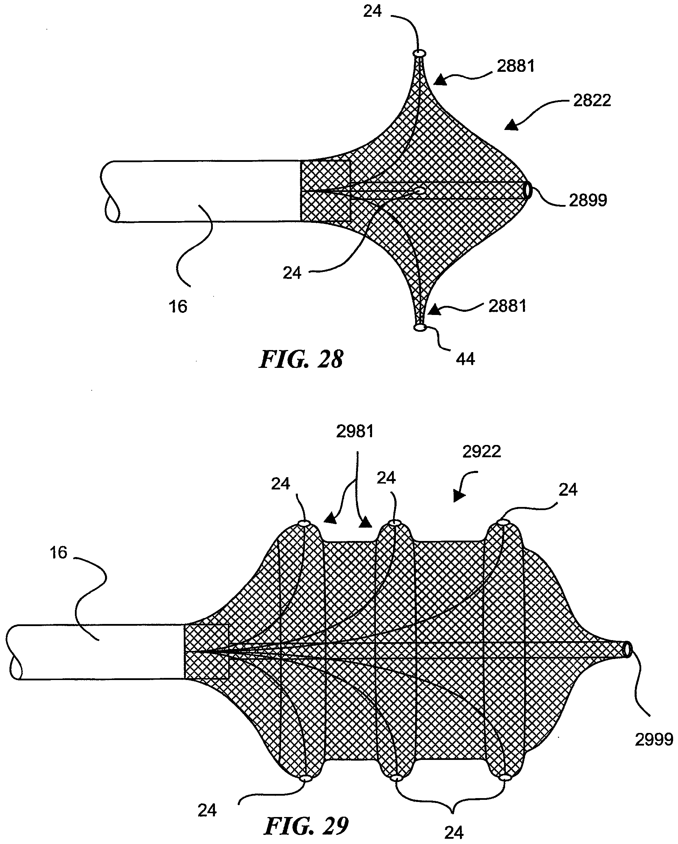

The mesh structure 22 can take on various shapes when expanded to control arterial surface contact and cooling fluid flow around the mesh structure 22. Figure 28, for example, illustrates an expanded mesh structure 2822 having mesh projections 2881 extending radially from the longitudinal axis of the elongated shaft 16. A plurality of mesh projections 2881 are disposed around the circumference of mesh structure 2822 and can comprise conical mesh projections terminating in electrodes 24 and permitting flow of cooling fluid therebetween or can comprise one or more annular rings or other shapes. Figure 29 likewise illustrates an expanded mesh structure 2922 having a plurality of spaced-apart annular mesh ribs 2981 formed in and protruding outwardly from a generally cylindrical mesh body. Mesh ribs 2981 can include energy delivery elements 24 mounted at outermost circumferential points to contact the renal artery wall while still allowing blood or other fluid to flow within and around the mesh structure 2922. The shapes of mesh points 2881 and mesh ribs 2981 can be heat-set into a generally cylindrical mesh body. In the illustrated embodiments, the mesh structures 2822, 2922 further include distal openings 2899, 2999 that permit passage of the guide wire 66.

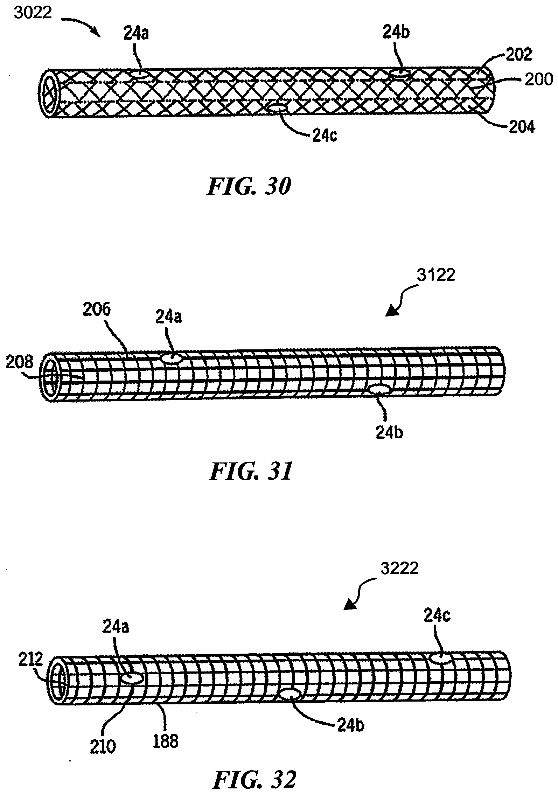

Alternatively, electrically isolated energy delivery elements 24 may be formed on a single mesh structure 22. Figure 30, for example, is a partial perspective view of a mesh structure 3022 formed from sections of insulated electrically conductive material 202 and 204 separated by a section 200 of electrically nonconductive material. Section 202 and section 204 are electrically isolated from one another and, therefore, may support respective electrically isolated energy delivery elements 24, which may be created by removing any insulating material to create a desired size, shape, and number of energy delivery elements 24. The sections 200, 202, and 204 may be joined together via adhesion or other suitable techniques.

Referring to Figure 31, a woven configuration of a mesh structure 3122 is depicted in which longitudinal fibers 206 (e.g., running axially along the length of the mesh structure 3122) are electrically conductive while the circumferential fibers 208 are electrically nonconductive. Because of the woven nature of the structure, the longitudinal fibers may be electrically isolated from one another because the circumferential fibers are electrically nonconductive. Alternatively, as shown in Figure 32, a mesh structure 3222 having the circumferential fibers 210 may be electrically conductive, while the longitudinal fibers 212 are electrically nonconductive. In both embodiments, the electrically conductive fibers (e.g., 206, 210) may be covered with an insulating polymer. Multiple energy delivery elements 24 that are electrically isolated from one another may be created by stripping away the insulating polymer from the electrically conductive fibers. In a particular embodiment, the insulating polymer coating the electrically conductive fibers may have a lower melt temperature relative to the electrically nonconductive fibers. By applying just enough heat to melt the insulating material from desired locations on the electrically conductive fibers, energy delivery elements 24 may be created without damaging the integrity of the electrically nonconductive fibers. In particular, energy delivery elements 24 may be formed in circumferential bands, either by stripping away insulating material from a latitudinal fiber 210 or by simply weaving uninsulated, electrically conductive fibers into the mesh structure 3222.

### D. Applying Energy to Tissue Via the Energy Delivery Element(s)

Referring back to Figure 1, the energy generator 26 may supply a continuous or pulsed RF electric field to the energy delivery element 24. Although a continuous delivery of RF energy is desirable, the application of RF energy in pulses may allow the application of relatively higher instantaneous power (e.g., higher power), longer or shorter total duration times, and/or better controlled intravascular renal neuromodulation therapy. Pulsed energy may also allow for the use of a smaller energy delivery element 24.

As previously discussed, energy delivery may be controlled and monitored via data collected with one or more sensors, such as temperature sensors (e.g., thermocouples, thermistors, etc.), impedance sensors, pressure sensors, optical sensors, flow sensors, chemical sensors, etc., which may be incorporated into or on the energy delivery element 24, the mesh structure 22, and/or in/on adjacent areas on the distal portion 20. A sensor may be incorporated into the energy delivery element 24 in a manner that specifies whether the sensor(s) are in contact with tissue at the treatment site and/or are facing blood flow. The ability to specify temperature sensor placement relative to tissue and blood flow is significant since a temperature gradient across the electrode from the side facing blood flow to the side in contact with the vessel wall may be, e.g., up to about 15°C (for platinum-iridium electrodes). In other embodiments including gold electrodes, this temperature gradient can be around, for example, 1-2°C. In still further embodiments, the temperature gradient can vary based, at least in part, on the electrode configuration/material. Significant gradients across the electrode in other sensed data (e.g., flow, pressure, impedance, etc.) also are expected.

The sensor(s) may, for example, be incorporated on or near the side of the energy delivery element 24 that contacts the vessel wall at the treatment site during power and energy delivery or may be incorporated on the opposing side of the energy delivery element 24 that faces blood flow during energy delivery, and/or may be incorporated within certain regions of the energy delivery element 24 (e.g., distal, proximal, quadrants, etc.). In some embodiments, multiple sensors may be provided at multiple positions along the energy delivery element 24 or mesh structure 22 and/or relative to blood flow. For example, a plurality of circumferentially and/or longitudinally spaced sensors may be provided. In one embodiment, a first sensor may face the vessel wall during treatment, and a second sensor may face blood flow.

Additionally or alternatively, various microsensors may be used to acquire data corresponding to the energy delivery element 24, the vessel wall and/or the blood flowing across the energy delivery element 24. For example, arrays of micro thermocouples and/or impedance sensors may be implemented to acquire data along the energy delivery element 24 or other parts of the treatment device. Sensor data may be acquired or monitored prior to, simultaneous with, or after the delivery of energy or in between pulses of energy, when applicable. The monitored data may be used in a feedback loop to better control therapy, e.g., to determine whether to continue or stop treatment, and it may facilitate controlled delivery of an increased or reduced power or a longer or shorter duration therapy.

### E. Blood Flow Around the Energy Delivery Element(s)

Non-target tissue may be protected by blood flow within the respective renal artery that serves as a conductive and/or convective heat sink that carries away excess thermal energy. For example, since blood flow is not blocked by the elongated shaft 16, the mesh structure 22, and the energy delivery element 24 it carries, the native circulation of blood in the respective renal artery serves to remove excess thermal energy from the non-target tissue and the energy delivery element. The removal of excess thermal energy by blood flow may also allow for treatments of higher power at lower surface temperatures, where more power may be delivered to the target tissue as thermal energy is carried away from the electrode and non-target tissue. In this way, intravascularly-delivered thermal energy heats target neural fibers located proximate to the vessel wall to modulate the target neural fibers, while blood flow within the respective renal artery protects non-target tissue of the vessel wall from excessive or undesirable thermal injury.

It may also be desirable to provide enhanced cooling by inducing additional native blood flow across the energy delivery element 24. For example, techniques and/or technologies may be implemented by the clinician to increase perfusion through the renal artery or to the energy delivery element 24 itself. These techniques include positioning partial occlusion elements (e.g., balloons) within upstream vascular bodies such as the aorta, or within a portion of the renal artery to improve flow across the energy delivery element.

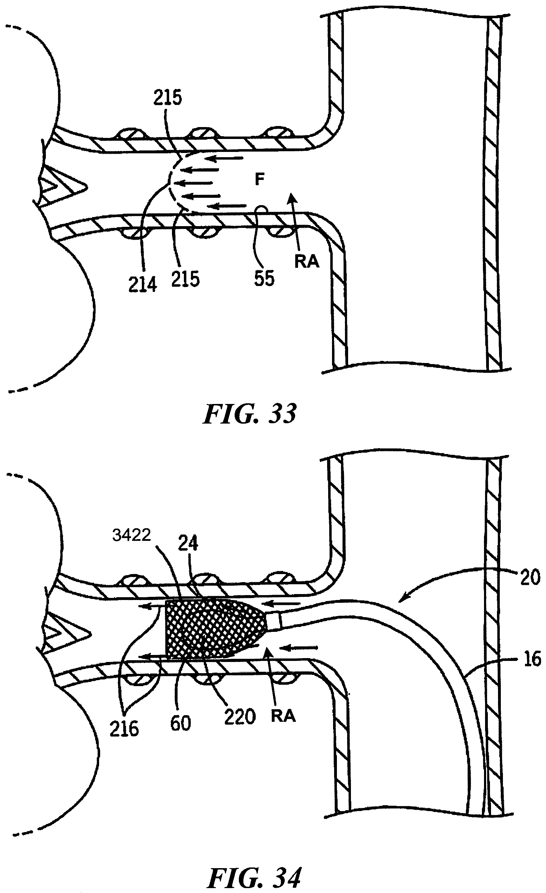

Figure 33, for example, illustrates hypothetical blood flow in a renal artery. Blood flow (F) is thought to be laminar, i.e., exhibit a gradient of flow velocities. In an area closest to the center of the artery, e.g., area 214, the blood flow velocity F may be faster relative to areas closer to the renal artery wall 55, e.g., areas 215. Accordingly, the blood flow F nearest the location of the energy delivery element 24 is relatively slow. Because cooling of the energy delivery element 24 is mediated by blood flow, improved cooling may be achieved by redirecting the blood flow F in the renal artery so that the blood flowing around the energy delivery element 24 is relatively faster.

Figure 34 illustrates an embodiment in which a fluid redirecting element 220 is positioned approximately within the center of the renal artery. Accordingly, the flowing blood, represented by arrows 216, including faster flowing blood, is redirected towards the energy delivery element 24. The fluid redirecting element may be any biocompatible material, such as PET, that is positioned to encourage blood flow towards the energy delivery element 24 on a mesh structure 3422.

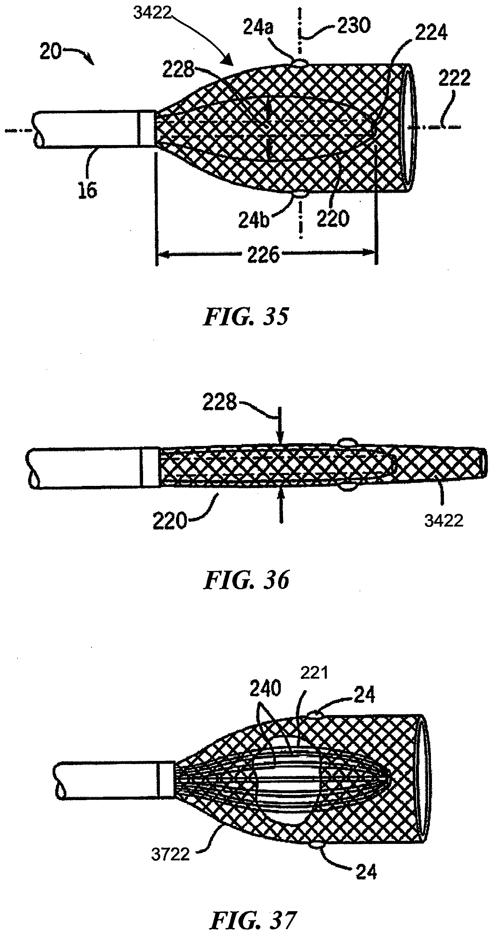

As shown in Figure 35, the fluid redirecting element 220 may extend from the distal end region 20 of the elongated shaft 16, generally along the axis 222 of the elongated shaft 16. For embodiments in which the guide wire 66 is used, the fluid redirecting element 220 may include an integral passage 224 sized and shaped to accommodate the guide wire 66. In addition, in some embodiments, the axial length 226 of the fluid redirecting element 220 may be at least 25%, at least 50%, or at least 75% of the axial length 80 of the mesh structure 3422 in the expanded configuration. In any case, in order to maximize redirected blood flow, the fluid redirecting element 220 may extend at least far enough into the mesh structure 3422 so that an imaginary axis 230 through the energy delivery element 24 (e.g., 24a or 24b) and orthogonal to the axis 222 intersects the fluid redirecting element 220. The diameter 228 of the fluid redirecting element 220 may be expandable such that in its unexpanded state it is generally compatible with insertion, repositioning, and removal of the mesh structure 3422 and in its expanded state it is configured to redirect blood flow toward areas closer to the renal artery wall, e.g., areas 215 of Figure 33. As shown in Figure 36, in a collapsed configuration, the mesh structure 3422 may conform to the shape of the fluid redirecting element 220. The diameter 228 may be slightly larger than, about equal to, or less than the diameter 61 of the elongated shaft. In one embodiment, the diameter 228 may be less than about 2 mm.

As shown in Figure 37, a fluid redirecting element 221 may include surface features 240, e.g., fluid dynamic features, for increasing blood flow past the energy delivery element 24 on a mesh structure 3722. The surface features 240 may include fins, grooves, channels, or rifling formed on the surface of the fluid redirecting element 220.

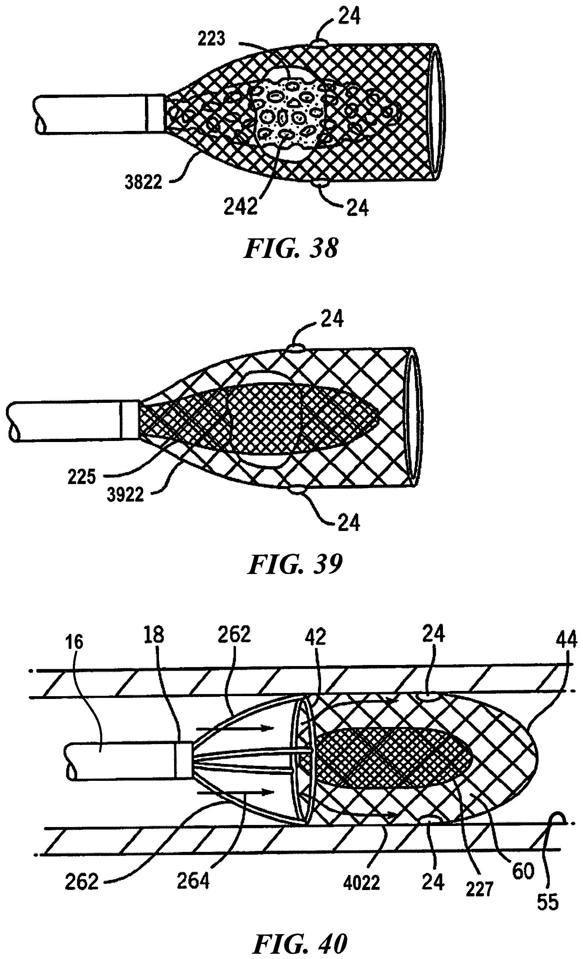

Figure 38 illustrates an embodiment in which a fluid redirecting element 223 is formed from a compressible material, such as open-cell foam. Pores 242 within the fluid redirecting element 223 allow blood to enter the fluid redirecting element 223 and facilitate its expansion within the renal artery. However, because the fluid redirecting element 223 may be compressed to a lower profile, its expandability may not interfere with insertion and/or removal of the treatment device. In this manner, the fluid redirecting element 223 may be configured to have an expanded diameter that is relatively larger than the diameter 61 of the elongated shaft. Alternatively, the fluid redirecting element 223 may take the form of an inflatable balloon, or an expandable mesh, as shown in Figure 39. In Figure 39, a fluid redirecting element 225 may be formed of a denser mesh relative to the mesh structure 3922.

A fluid redirecting element may also be used in conjunction with an umbrella-type or parachute-type mesh structure 22. For example, as illustrated in Figure 40, the proximal end 42 of the mesh structure 4022 may be axially separated and coupled to the elongated shaft 16 via wires 262. A fluid redirecting element 227 (e.g., a solid, foam, or expandable mesh) may be disposed along a wire 262 to redirect blood flow 264 towards the energy delivery element 24.

In addition, or as an alternative, to passively utilizing blood flow as a heat sink, active cooling may be provided to remove excess thermal energy and protect non-target tissues. For example, a thermal fluid infusate may be injected, infused, or otherwise delivered into the vessel in an open circuit system. Thermal fluid infusates used for active cooling may, for example, include (room temperature or chilled) saline or some other biocompatible fluid. The thermal fluid infusate(s) may, for example, be introduced through the treatment device 12 via one or more infusion lumens and/or ports. When introduced into the bloodstream, the thermal fluid infusate(s) may, for example, be introduced through a guide catheter at a location upstream from the energy delivery element 24 or at other locations relative to the tissue for which protection is sought. In a particular embodiment fluid infusate is injected through a lumen into internal space 60 so as to flow through interstitial spaces between filaments 58 and around energy delivery elements 24. The delivery of a thermal fluid infusate in the vicinity of the treatment site (via an open circuit system and/or via a closed circuit system) may, for example, allow for the application of increased/higher power treatment, may allow for the maintenance of lower temperature at the vessel wall during energy delivery, may facilitate the creation of deeper or larger lesions, may facilitate a reduction in treatment time, may allow for the use of a smaller electrode size, or a combination thereof.

Accordingly, the treatment device 12 may include features for an open circuit cooling system, such as a lumen in fluid communication with a source of infusate and a pumping mechanism (e.g., manual injection or a motorized pump) for injection or infusion of saline or some other biocompatible thermal fluid infusate from outside the patient, through elongated shaft 16 and towards the energy delivery element 24 into the patient's bloodstream during energy delivery. In addition, the distal end region 20 of the elongated shaft 16 may include one or more ports for injection or infusion of saline directly at the treatment site.

### III. Use of the System

### A. Intravascular Delivery. Deflection and Placement of the Treatment Device

As mentioned previously, any one of the embodiments of the treatment devices described herein may be delivered using over-the-wire ("OTW") or rapid exchange ("RX") techniques. When delivered in this manner, the elongated shaft 16 includes a passage or lumen accommodating passage of a guide wire. Alternatively, any one of the treatment devices described herein may be deployed using a conventional guide catheter or pre-curved renal guide catheter (e.g., as shown in Figure 8). When using a guide catheter, the femoral artery is exposed and cannulated at the base of the femoral triangle, using conventional techniques. In one approach, a guide wire may be inserted through the access site and passed using image guidance through the femoral artery, into the iliac artery and aorta, and into either the left or right renal artery. A guide catheter may be passed over the guide wire into the accessed renal artery. The guide wire may then be removed. Alternatively, a renal guide catheter, which is specifically shaped and configured to access a renal artery, may be used instead of using a guide wire. Still alternatively, the treatment device may be routed from the femoral artery to the renal artery using angiographic guidance and without the need of a guide catheter.

When a guide catheter is used, at least three delivery approaches may be implemented. In one approach, one or more of the aforementioned delivery techniques may be used to position a guide catheter within the renal artery just distal to the entrance of the renal artery. The treatment device is then routed via the guide catheter into the renal artery. Once the treatment device is properly positioned within the renal artery, the guide catheter can be retracted from the renal artery into the abdominal aorta. In this approach, the guide catheter should be sized and configured to accommodate passage of the treatment device. For example, a 6 French guide catheter may be used.

In a second approach, a first guide catheter is placed at the entrance of the renal artery (with or without a guide wire). A second guide catheter (also called a delivery sheath) is passed via the first guide catheter (with or without the assistance of a guide wire) into the renal artery. The treatment device is then routed via the second guide catheter into the renal artery. Once the treatment device is properly positioned within the renal artery the second guide catheter is retracted, leaving the first guide catheter at the entrance to the renal artery. In this approach the first and second guide catheters should be sized and configured to accommodate passage of the second guide catheter within the first guide catheter (i.e., the inner diameter of the first guide catheter should be greater than the outer diameter of the second guide catheter). For example, a 8 French guide catheter may be used for the first guide catheter, and 5 French guide catheter may be used for the second guide catheter.

In a third approach, a renal guide catheter may be positioned within the abdominal aorta just proximal to the entrance of the renal artery. Any one of the treatment devices described herein may be passed through the guide catheter and into the accessed renal artery. The elongated shaft makes atraumatic passage through the guide catheter, in response to forces applied to the elongated shaft 16 through the handle assembly 34.

### B. Control of Applied Energy

With the treatments disclosed herein for delivering therapy to target tissue, it may be beneficial for energy to be delivered to the target neural structures in a controlled manner. The controlled delivery of energy will allow the zone of thermal treatment to extend into the renal fascia while reducing undesirable energy delivery or thermal effects to the vessel wall. A controlled delivery of energy may also result in a more consistent, predictable and efficient overall treatment. Accordingly, as noted previously, the energy generator 26 can include a processor-based control including a memory with instructions for executing an algorithm 30 (see Figure 1) for controlling the delivery of power and energy to the energy delivery device 24. The algorithm 30, a representative embodiment of which is shown in Figure 41, may be implemented as a conventional computer program for execution by a processor coupled to the energy generator 26. A clinician using step-by-step instructions may also implement the algorithm 30 manually.

The operating parameters monitored in accordance with the algorithm may include, for example, temperature, time, impedance, power, flow velocity, volumetric flow rate, blood pressure, heart rate, etc. Discrete values in temperature may be used to trigger changes in power or energy delivery. For example, high values in temperature (e.g., 85°C) could indicate increased risk of thrombosis, etc., in which case the algorithm may decrease or stop the power and energy delivery to prevent undesirable thermal effects to target or non-target tissue. Time additionally or alternatively may be used to prevent undesirable thermal alteration to non-target tissue. For each treatment, a set time (e.g., 2 minutes) is checked to prevent indefinite delivery of power.

Impedance may be used to measure tissue changes. Impedance indicates the electrical property of the treatment site. In thermal inductive embodiments, when electric field is applied to the treatment site, the impedance will decrease as the tissue become less resistive to current flow. If too much energy is applied, tissue desiccation or coagulation may occur near the electrode. When tissue at the treatment site becomes desiccated or decreases in water content, it becomes less electrically conductive, resulting in an overall increase in sensed impedance. When high impedance coagulum forms on the surface of an electrode, the covered area becomes insulated to some degree and active surface area is effectively decreased. Impedance is inversely proportional to electrode surface area. Therefore, insulating part of the electrode and decreasing active surface area can result in an increase in impedance. An increase in tissue impedance may be indicative or predictive of undesirable thermal alteration to target or non-target tissue. In other embodiments, the impedance value may be used to assess contact of the energy delivery element 24 with the tissue. For a single electrode configuration, a relatively high, stable impedance value may be indicative of good contact. For a multiple electrode configurations, relatively high, stable impedance values on both electrodes and a relatively small and stable difference in impedance values may be indicative of good contact with the tissue. Accordingly, impedance information may be provided to a downstream monitor, which in turn may provide an indication to a clinician related to the quality of the energy delivery element 24 contact with the tissue. Additionally or alternatively, power is an effective parameter to monitor in controlling the delivery of therapy. Power is a function of voltage and current. The algorithm may tailor the voltage and/or current to achieve a desired power.

Derivatives of the aforementioned parameters (e.g., rates of change) also may be used to trigger changes in power or energy delivery. For example, the rate of change in temperature could be monitored such that power output is reduced in the event that a sudden rise in temperature is detected. Likewise, the rate of change of impedance could be monitored such that power output is reduced in the event that a sudden rise in impedance is detected.

As seen in Figure 41, when a clinician initiates treatment (e.g., via the foot pedal), the control algorithm 30 includes instructions to the generator 26 to gradually adjust its power output to a first power level P₁ (e.g., 5 watts) over a first time period t₁ (e.g., 15 seconds). The power increase during the first time period is generally linear. As a result, the generator 26 increases its power output at a generally constant rate of P₁/t₁. Alternatively, the power increase may be non-linear (e.g., exponential or parabolic) with a variable rate of increase. Once P₁ and t₁ are achieved, the algorithm may hold at P₁ until a new time t₂ for a predetermined period of time t₂ - t₁ (e.g., 3 seconds). At t₂ power is increased by a predetermined increment (e.g., 1 watt) to P₂ over a predetermined period of time, t₃ - t₂ (e.g., 1 second). This power ramp in predetermined increments of about 1 watt over predetermined periods of time may continue until a maximum power P_{MAX} is achieved or some other condition is satisfied. In one embodiment, P_{MAX} is 8 watts. In another embodiment P_{MAX} is 10 watts. Optionally, the power may be maintained at the maximum power P_{MAX} for a desired period of time or up to the desired total treatment time (e.g., up to about 120 seconds).

In Figure 41, algorithm 30 illustratively includes a power-control algorithm. However, it should be understood that algorithm 30 alternatively may include a temperature-control algorithm. For example, power may be gradually increased until a desired temperature (or temperatures) is obtained for a desired duration (durations). In another embodiment, a combination power-control and temperature-control algorithm may be provided. In still further embodiments, the algorithm 30 may include additional features and/or have different parameters.

As discussed, the algorithm 30 includes monitoring certain operating parameters (e.g., temperature, time, impedance, power, flow velocity, volumetric flow rate, blood pressure, heart rate, etc.). The operating parameters may be monitored continuously or periodically. The algorithm 30 checks the monitored parameters against predetermined parameter profiles to determine whether the parameters individually or in combination fall within the ranges set by the predetermined parameter profiles. If the monitored parameters fall within the ranges set by the predetermined parameter profiles, then treatment may continue at the commanded power output. If monitored parameters fall outside the ranges set by the predetermined parameter profiles, the algorithm 30 adjusts the commanded power output accordingly. For example, if a target temperature (e.g., 65°C) is achieved, then power delivery is kept constant until the total treatment time (e.g., 120 seconds) has expired. If a first temperature threshold (e.g., 70°C) is achieved or exceeded, then power is reduced in predetermined increments (e.g., 0.5 watts, 1.0 watts, etc.) until a target temperature is achieved. If a second power threshold (e.g., 85°C) is achieved or exceeded, thereby indicating an undesirable condition, then power delivery may be terminated. The system may be equipped with various audible and visual alarms to alert the operator of certain conditions.

The following is a non-exhaustive list of events under which algorithm 30 may adjust and/or terminate/discontinue the commanded power output:
(1) The measured temperature exceeds a maximum temperature threshold (e.g., about 70 to about 85°C.).
(2) The average temperature derived from the measured temperature exceeds an average temperature threshold (e.g., about 65°C.).
(3) The rate of change of the measured temperature exceeds a rate of change threshold.
(4) The temperature rise over a period of time is below a minimum temperature change threshold while the generator 26 has non-zero output. Poor contact between the energy delivery element 24 and the arterial wall may cause such a condition.
(5) A measured impedance exceeds an impedance threshold (e.g., <20 Ohms, or >500 Ohms).
(6) A measured impedance exceeds a relative threshold (e.g., impedance decreases from a starting or baseline value and then rises above this baseline value)
(7) A measured power exceeds a power threshold (e.g., >8 Watts or >10 Watts).
(8) A measured duration of power delivery exceeds a time threshold (e.g., >120 seconds).

It should be understood that the foregoing list of parameters are merely provided as examples. In other embodiments, the algorithm 30 may include a variety of different parameters. For example, different electrode designs/configurations can result in changes to the operating parameters.

Advantageously, the magnitude of maximum power delivered during renal neuromodulation treatment in accordance with the present technology may be relatively low (e.g., less than about 15 Watts, for example, less than about 10 Watts or less than about 8 Watts) as compared, for example, to the power levels utilized in electrophysiology treatments to achieve cardiac tissue ablation (e.g., power levels greater than about 15 Watts, for example, greater than about 30 Watts). Since relatively low power levels may be utilized to achieve such renal neuromodulation, the flow rate and/or total volume of intravascular infusate injection needed to maintain the energy delivery element and/or non-target tissue at or below a desired temperature during power delivery (e.g., at or below about 50°C, for example, at or below about 45°C) also may be relatively lower than would be required at the higher power levels used, for example, in electrophysiology treatments (e.g., power levels above about 15 Watts). In embodiments in which active cooling is used, the relative reduction in flow rate and/or total volume of intravascular infusate infusion advantageously may facilitate the use of intravascular infusate in higher risk patient groups that would be contraindicated were higher power levels and, thus, correspondingly higher infusate rates/volumes utilized (e.g., patients with heart disease, heart failure, renal insufficiency and/or diabetes mellitus).

In embodiments comprising relatively large energy delivery elements 24 (e.g., such as the embodiment shown in Figure 25 in which the mesh structure is electrically conductive and all or a large portion of the mesh structure is uninsulated) larger magnitudes of power may be delivered during renal neuromodulation treatment in accordance with the present technology. For example, power levels of about 30 W to 40 W may be sufficient with relatively large energy delivery elements that may spread the power over a large area.

### IV. Prepackaged Kit for Distribution, Transport and Sale of the Disclosed Apparatuses and Systems

As shown in Figure 42, one or more components of the system 10 shown in Figure 1 may be packaged together in a kit 276 for convenient delivery to and use by the customer/clinical operator. Components suitable for packaging include, the treatment device 12, the cable 28 for connecting the treatment device 12 to the energy generator 26, the neutral or dispersive electrode 38, and/or one or more guide catheters (e.g., a renal guide catheter). Cable 28 may also be integrated into the treatment device 12 such that both components are packaged together. Each component may have its own sterile packaging (for components requiring sterilization) or the components may have dedicated sterilized compartments within the kit packaging. This kit may also include step-by-step instructions 280 for use that provide the operator with technical product features and operating instructions for using the system 10 and treatment device 12, including all methods of insertion, delivery, placement, and use of the treatment device 12 disclosed herein.

### V. Additional Clinical Uses of the Disclosed Technology

Although certain embodiments of the present techniques relate to at least partially denervating a kidney of a patient to block afferent and/or efferent neural communication from within a renal blood vessel (e.g., renal artery), the apparatuses, methods and systems described herein may also be used for other intravascular treatments. For example, the aforementioned catheter system, or select aspects of such system, may be placed in other peripheral blood vessels to deliver energy and/or electric fields to achieve a neuromodulatory affect by altering nerves proximate to these other peripheral blood vessels. There are a number of arterial vessels arising from the aorta which travel alongside a rich collection of nerves to target organs. Utilizing the arteries to access and modulate these nerves may have clear therapeutic potential in a number of disease states. Some examples include the nerves encircling the celiac trunk, superior mesenteric artery, and inferior mesenteric artery.

Sympathetic nerves proximate to or encircling the arterial blood vessel known as the celiac trunk may pass through the celiac ganglion and follow branches of the celiac trunk to innervate the stomach, small intestine, abdominal blood vessels, liver, bile ducts, gallbladder, pancreas, adrenal glands, and kidneys. Modulating these nerves in whole (or in part via selective modulation) may enable treatment of conditions including, but not limited to, diabetes, pancreatitis, obesity, hypertension, obesity related hypertension, hepatitis, hepatorenal syndrome, gastric ulcers, gastric motility disorders, irritable bowel syndrome, and autoimmune disorders such as Crohn's disease.

Sympathetic nerves proximate to or encircling the arterial blood vessel known as the inferior mesenteric artery may pass through the inferior mesenteric ganglion and follow branches of the inferior mesenteric artery to innervate the colon, rectum, bladder, sex organs, and external genitalia. Modulating these nerves in whole (or in part via selective modulation) may enable treatment of conditions including, but not limited to, GI motility disorders, colitis, urinary retention, hyperactive bladder, incontinence, infertility, polycystic ovarian syndrome, premature ejaculation, erectile dysfunction, dyspareunia, and vaginismus.

While arterial access and treatments received have been provided herein, the disclosed apparatuses, methods and systems may also be used to deliver treatment from within a peripheral vein or lymphatic vessel.

### VI. Additional Discussion of Pertinent Anatomy and Physiology

The following discussion provides further details regarding pertinent patient anatomy and physiology. This section is intended to supplement and expand upon the previous discussion regarding the relevant anatomy and physiology, and to provide additional context regarding the disclosed technology and the therapeutic benefits associated with renal denervation. For example, as mentioned previously, several properties of the renal vasculature may inform the design of treatment devices and associated methods for achieving renal neuromodulation via intravascular access, and impose specific design requirements for such devices. Specific design requirements may include accessing the renal artery, facilitating stable contact between the energy delivery element(s) of such devices and a luminal surface or wall of the renal artery, and/or effectively modulating the renal nerves with the neuromodulatory apparatus.

### A. The Sympathetic Nervous System

The Sympathetic Nervous System (SNS) is a branch of the autonomic nervous system along with the enteric nervous system and parasympathetic nervous system. It is always active at a basal level (called sympathetic tone) and becomes more active during times of stress. Like other parts of the nervous system, the sympathetic nervous system operates through a series of interconnected neurons. Sympathetic neurons are frequently considered part of the peripheral nervous system (PNS), although many lie within the central nervous system (CNS). Sympathetic neurons of the spinal cord (which is part of the CNS) communicate with peripheral sympathetic neurons via a series of sympathetic ganglia. Within the ganglia, spinal cord sympathetic neurons join peripheral sympathetic neurons through synapses. Spinal cord sympathetic neurons are therefore called presynaptic (or preganglionic) neurons, while peripheral sympathetic neurons are called postsynaptic (or postganglionic) neurons.

At synapses within the sympathetic ganglia, preganglionic sympathetic neurons release acetylcholine, a chemical messenger that binds and activates nicotinic acetylcholine receptors on postganglionic neurons. In response to this stimulus, postganglionic neurons principally release noradrenaline (norepinephrine). Prolonged activation may elicit the release of adrenaline from the adrenal medulla.

Once released, norepinephrine and epinephrine bind adrenergic receptors on peripheral tissues. Binding to adrenergic receptors causes a neuronal and hormonal response. The physiologic manifestations include pupil dilation, increased heart rate, occasional vomiting, and increased blood pressure. Increased sweating is also seen due to binding of cholinergic receptors of the sweat glands.

The sympathetic nervous system is responsible for up- and down-regulating many homeostatic mechanisms in living organisms. Fibers from the SNS innervate tissues in almost every organ system, providing at least some regulatory function to things as diverse as pupil diameter, gut motility, and urinary output. This response is also known as *sympatho-adrenal response* of the body, as the preganglionic sympathetic fibers that end in the adrenal medulla (but also all other sympathetic fibers) secrete acetylcholine, which activates the secretion of adrenaline (epinephrine) and to a lesser extent noradrenaline (norepinephrine). Therefore, this response that acts primarily on the cardiovascular system is mediated directly via impulses transmitted through the sympathetic nervous system and indirectly via catecholamines secreted from the adrenal medulla.

Science typically looks at the SNS as an automatic regulation system, that is, one that operates without the intervention of conscious thought. Some evolutionary theorists suggest that the sympathetic nervous system operated in early organisms to maintain survival as the sympathetic nervous system is responsible for priming the body for action. One example of this priming is in the moments before waking, in which sympathetic outflow spontaneously increases in preparation for action.

### 1. The Sympathetic Chain

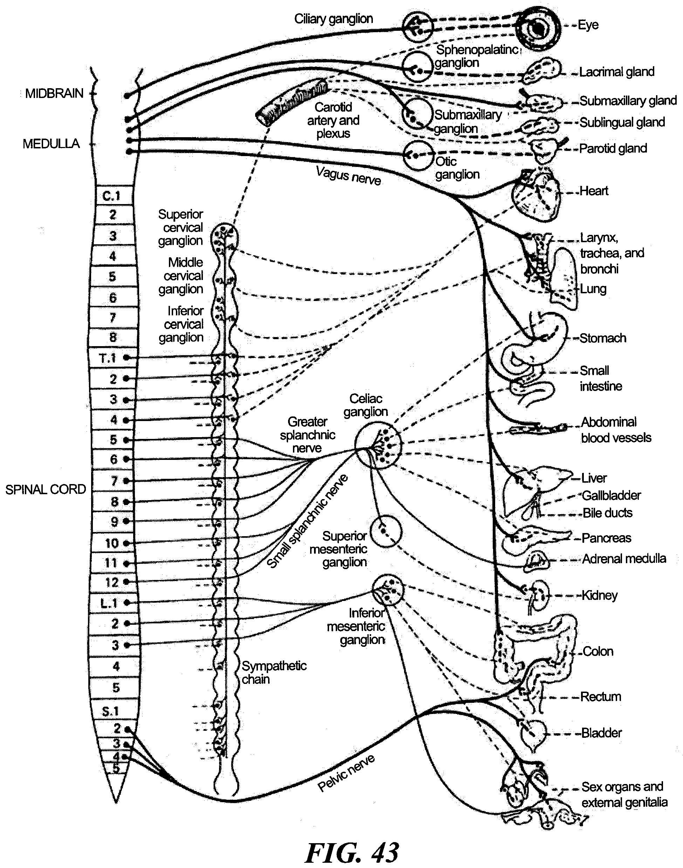

As shown in Figure 43, the SNS provides a network of nerves that allows the brain to communicate with the body. Sympathetic nerves originate inside the vertebral column, toward the middle of the spinal cord in the intermediolateral cell column (or lateral horn), beginning at the first thoracic segment of the spinal cord and are thought to extend to the second or third lumbar segments. Because its cells begin in the thoracic and lumbar regions of the spinal cord, the SNS is said to have a *thoracolumbar outflow.* Axons of these nerves leave the spinal cord through the anterior rootlet/root. They pass near the spinal (sensory) ganglion, where they enter the anterior rami of the spinal nerves. However, unlike somatic innervation, they quickly separate out through white rami connectors which connect to either the paravertebral (which lie near the vertebral column) or prevertebral (which lie near the aortic bifurcation) ganglia extending alongside the spinal column.

In order to reach the target organs and glands, the axons should travel long distances in the body, and, to accomplish this, many axons relay their message to a second cell through synaptic transmission. The ends of the axons link across a space, the synapse, to the dendrites of the second cell. The first cell (the presynaptic cell) sends a neurotransmitter across the synaptic cleft where it activates the second cell (the postsynaptic cell). The message is then carried to the final destination.

In the SNS and other components of the peripheral nervous system, these synapses are made at sites called ganglia. The cell that sends its fiber is called a preganglionic cell, while the cell whose fiber leaves the ganglion is called a postganglionic cell. As mentioned previously, the preganglionic cells of the SNS are located between the first thoracic (T1) segment and third lumbar (L3) segments of the spinal cord. Postganglionic cells have their cell bodies in the ganglia and send their axons to target organs or glands.

The ganglia include not just the sympathetic trunks but also the cervical ganglia (superior, middle and inferior), which sends sympathetic nerve fibers to the head and thorax organs, and the celiac and mesenteric ganglia (which send sympathetic fibers to the gut).

### 2. Innervation of the Kidneys

As Figure 44 shows, the kidney is innervated by the renal plexus RP, which is intimately associated with the renal artery. The renal plexus RP is an autonomic plexus that surrounds the renal artery and is embedded within the adventitia of the renal artery. The renal plexus RP extends along the renal artery until it arrives at the substance of the kidney. Fibers contributing to the renal plexus RP arise from the celiac ganglion, the superior mesenteric ganglion, the aorticorenal ganglion and the aortic plexus. The renal plexus RP, also referred to as the renal nerve, is predominantly comprised of sympathetic components. There is no (or at least very minimal) parasympathetic innervation of the kidney.

Preganglionic neuronal cell bodies are located in the intermediolateral cell column of the spinal cord. Preganglionic axons pass through the paravertebral ganglia (they do not synapse) to become the lesser splanchnic nerve, the least splanchnic nerve, first lumbar splanchnic nerve, second lumbar splanchnic nerve, and travel to the celiac ganglion, the superior mesenteric ganglion, and the aorticorenal ganglion. Postganglionic neuronal cell bodies exit the celiac ganglion, the superior mesenteric ganglion, and the aorticorenal ganglion to the renal plexus RP and are distributed to the renal vasculature.

### 3. Renal Sympathetic Neural Activity

Messages travel through the SNS in a bidirectional flow. Efferent messages may trigger changes in different parts of the body simultaneously. For example, the sympathetic nervous system may accelerate heart rate; widen bronchial passages; decrease motility (movement) of the large intestine; constrict blood vessels; increase peristalsis in the esophagus; cause pupil dilation, piloerection (goose bumps) and perspiration (sweating); and raise blood pressure. Afferent messages carry signals from various organs and sensory receptors in the body to other organs and, particularly, the brain.

Hypertension, heart failure and chronic kidney disease are a few of many disease states that result from chronic activation of the SNS, especially the renal sympathetic nervous system. Chronic activation of the SNS is a maladaptive response that drives the progression of these disease states. Pharmaceutical management of the renin-angiotensin-aldosterone system (RAAS) has been a longstanding, but somewhat ineffective, approach for reducing overactivity of the SNS.

As mentioned above, the renal sympathetic nervous system has been identified as a major contributor to the complex pathophysiology of hypertension, states of volume overload (such as heart failure), and progressive renal disease, both experimentally and in humans. Studies employing radiotracer dilution methodology to measure overflow of norepinephrine from the kidneys to plasma revealed increased renal norepinephrine (NE) spillover rates in patients with essential hypertension, particularly so in young hypertensive subjects, which in concert with increased NE spillover from the heart, is consistent with the hemodynamic profile typically seen in early hypertension and characterized by an increased heart rate, cardiac output, and renovascular resistance. It is now known that essential hypertension is commonly neurogenic, often accompanied by pronounced sympathetic nervous system overactivity.

Activation of cardiorenal sympathetic nerve activity is even more pronounced in heart failure, as demonstrated by an exaggerated increase of NE overflow from the heart and the kidneys to plasma in this patient group. In line with this notion is the recent demonstration of a strong negative predictive value of renal sympathetic activation on all-cause mortality and heart transplantation in patients with congestive heart failure, which is independent of overall sympathetic activity, glomerular filtration rate, and left ventricular ejection fraction. These findings support the notion that treatment regimens that are designed to reduce renal sympathetic stimulation have the potential to improve survival in patients with heart failure.

Both chronic and end stage renal disease are characterized by heightened sympathetic nervous activation. In patients with end stage renal disease, plasma levels of norepinephrine above the median have been demonstrated to be predictive for both all-cause death and death from cardiovascular disease. This is also true for patients suffering from diabetic or contrast nephropathy. There is compelling evidence suggesting that sensory afferent signals originating from the diseased kidneys are major contributors to initiating and sustaining elevated central sympathetic outflow in this patient group; this facilitates the occurrence of the well known adverse consequences of chronic sympathetic over activity, such as hypertension, left ventricular hypertrophy, ventricular arrhythmias, sudden cardiac death, insulin resistance, diabetes, and metabolic syndrome.

### (i) Renal Sympathetic Efferent Activity

Sympathetic nerves to the kidneys terminate in the blood vessels, the juxtaglomerular apparatus and the renal tubules. Stimulation of the renal sympathetic nerves causes increased renin release, increased sodium (Na+) reabsorption, and a reduction of renal blood flow. These components of the neural regulation of renal function are considerably stimulated in disease states characterized by heightened sympathetic tone and clearly contribute to the rise in blood pressure in hypertensive patients. The reduction of renal blood flow and glomerular filtration rate as a result of renal sympathetic efferent stimulation is likely a cornerstone of the loss of renal function in cardio-renal syndrome, which is renal dysfunction as a progressive complication of chronic heart failure, with a clinical course that typically fluctuates with the patient's clinical status and treatment. Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin II and aldosterone activation consequent to renin release) and diuretics (intended to counter the renal sympathetic mediated sodium and water retention). However, the current pharmacologic strategies have significant limitations including limited efficacy, compliance issues, side effects and others.

### (ii) Renal Sensory Afferent Nerve Activity

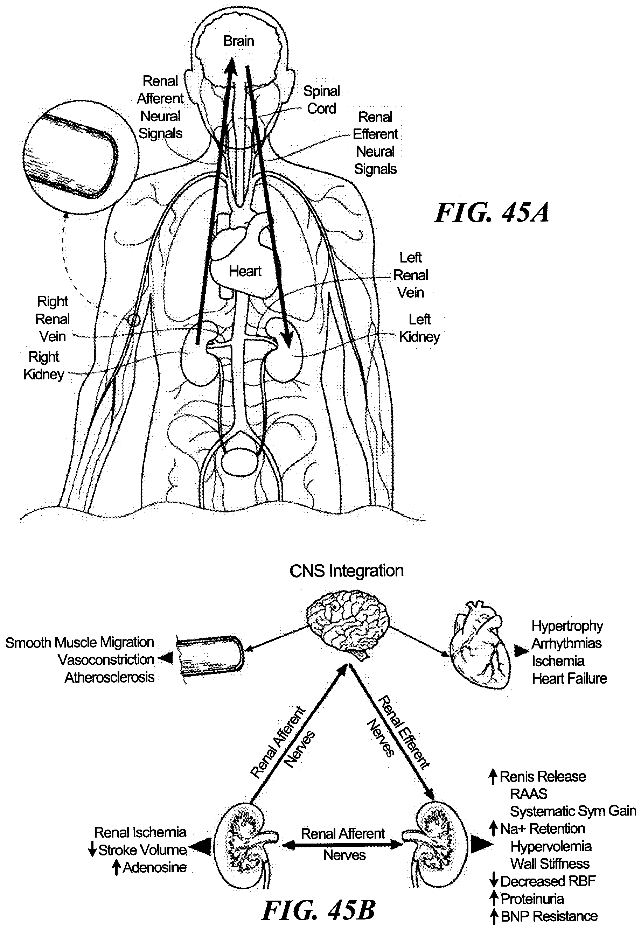

The kidneys communicate with integral structures in the central nervous system via renal sensory afferent nerves. Several forms of "renal injury" may induce activation of sensory afferent signals. For example, renal ischemia, reduction in stroke volume or renal blood flow, or an abundance of adenosine enzyme may trigger activation of afferent neural communication. As shown in Figures 45A and 45B, this afferent communication might be from the kidney to the brain or might be from one kidney to the other kidney (via the central nervous system). These afferent signals are centrally integrated and may result in increased sympathetic outflow. This sympathetic drive is directed towards the kidneys, thereby activating the RAAS and inducing increased renin secretion, sodium retention, volume retention and vasoconstriction. Central sympathetic over activity also impacts other organs and bodily structures innervated by sympathetic nerves such as the heart and the peripheral vasculature, resulting in the described adverse effects of sympathetic activation, several aspects of which also contribute to the rise in blood pressure.

The physiology therefore suggests that (i) modulation of tissue with efferent sympathetic nerves will reduce inappropriate renin release, salt retention, and reduction of renal blood flow, and that (ii) modulation of tissue with afferent sensory nerves will reduce the systemic contribution to hypertension and other disease states associated with increased central sympathetic tone through its direct effect on the posterior hypothalamus as well as the contralateral kidney. In addition to the central hypotensive effects of afferent renal denervation, a desirable reduction of central sympathetic outflow to various other sympathetically innervated organs such as the heart and the vasculature is anticipated.

### B. Additional Clinical Benefits of Renal Denervation

As provided above, renal denervation is likely to be valuable in the treatment of several clinical conditions characterized by increased overall and particularly renal sympathetic activity such as hypertension, metabolic syndrome, insulin resistance, diabetes, left ventricular hypertrophy, chronic end stage renal disease, inappropriate fluid retention in heart failure, cardio-renal syndrome, and sudden death. Since the reduction of afferent neural signals contributes to the systemic reduction of sympathetic tone/drive, renal denervation might also be useful in treating other conditions associated with systemic sympathetic hyperactivity. Accordingly, renal denervation may also benefit other organs and bodily structures innervated by sympathetic nerves, including those identified in Figure 43. For example, as previously discussed, a reduction in central sympathetic drive may reduce the insulin resistance that afflicts people with metabolic syndrome and Type II diabetics. Additionally, patients with osteoporosis are also sympathetically activated and might also benefit from the down regulation of sympathetic drive that accompanies renal denervation.

### C. Achieving Intravascular Access to the Renal Artery

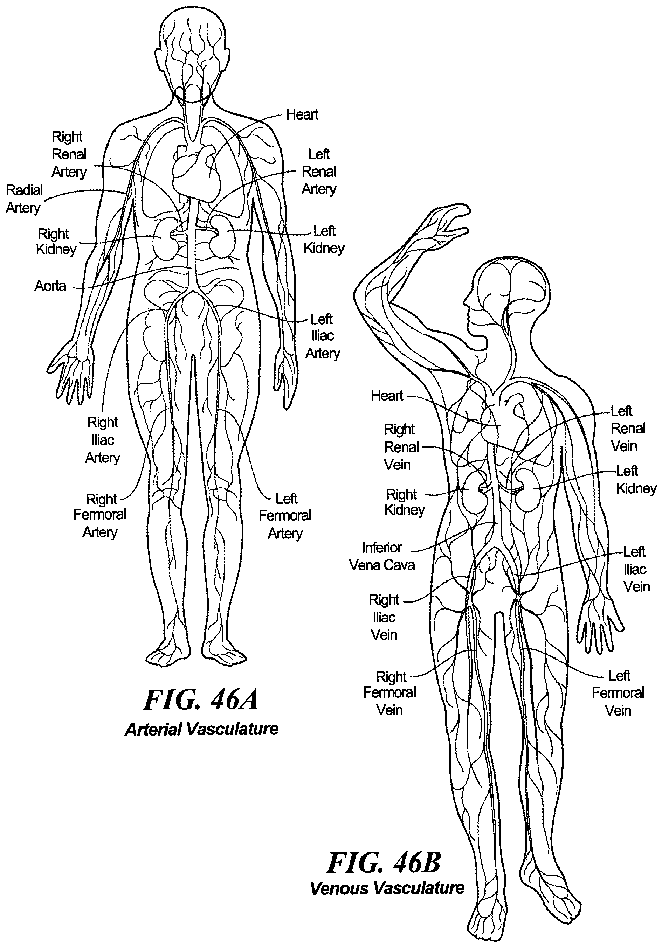

In accordance with the present technology, neuromodulation of a left and/or right renal plexus RP, which is intimately associated with a left and/or right renal artery, may be achieved through intravascular access. As Figure 46A shows, blood moved by contractions of the heart is conveyed from the left ventricle of the heart by the aorta. The aorta descends through the thorax and branches into the left and right renal arteries. Below the renal arteries, the aorta bifurcates at the left and right iliac arteries. The left and right iliac arteries descend, respectively, through the left and right legs and join the left and right femoral arteries.

As Figure 46B shows, the blood collects in veins and returns to the heart, through the femoral veins into the iliac veins and into the inferior vena cava. The inferior vena cava branches into the left and right renal veins. Above the renal veins, the inferior vena cava ascends to convey blood into the right atrium of the heart. From the right atrium, the blood is pumped through the right ventricle into the lungs, where it is oxygenated. From the lungs, the oxygenated blood is conveyed into the left atrium. From the left atrium, the oxygenated blood is conveyed by the left ventricle back to the aorta.

As will be described in greater detail later, the femoral artery may be accessed and cannulated at the base of the femoral triangle just inferior to the midpoint of the inguinal ligament. A catheter may be inserted percutaneously into the femoral artery through this access site, passed through the iliac artery and aorta, and placed into either the left or right renal artery. This comprises an intravascular path that offers minimally invasive access to a respective renal artery and/or other renal blood vessels.

The wrist, upper arm, and shoulder region provide other locations for introduction of catheters into the arterial system. For example, catheterization of either the radial, brachial, or axillary artery may be utilized in select cases. Catheters introduced via these access points may be passed through the subclavian artery on the left side (or via the subclavian and brachiocephalic arteries on the right side), through the aortic arch, down the descending aorta and into the renal arteries using standard angiographic technique.

### D. Properties and Characteristics of the Renal Vasculature

Since neuromodulation of a left and/or right renal plexus RP may be achieved in accordance with the present technology through intravascular access, properties and characteristics of the renal vasculature may impose constraints upon and/or inform the design of apparatus, systems, and methods for achieving such renal neuromodulation. Some of these properties and characteristics may vary across the patient population and/or within a specific patient across time, as well as in response to disease states, such as hypertension, chronic kidney disease, vascular disease, end-stage renal disease, insulin resistance, diabetes, metabolic syndrome, etc. These properties and characteristics, as explained herein, may have bearing on the efficacy of the procedure and the specific design of the intravascular device. Properties of interest may include, for example, material/mechanical, spatial, fluid dynamic/hemodynamic and/or thermodynamic properties.

As discussed previously, a catheter may be advanced percutaneously into either the left or right renal artery via a minimally invasive intravascular path. However, minimally invasive renal arterial access may be challenging, for example, because as compared to some other arteries that are routinely accessed using catheters, the renal arteries are often extremely tortuous, may be of relatively small diameter, and/or may be of relatively short length. Furthermore, renal arterial atherosclerosis is common in many patients, particularly those with cardiovascular disease. Renal arterial anatomy also may vary significantly from patient to patient, which further complicates minimally invasive access. Significant inter-patient variation may be seen, for example, in relative tortuosity, diameter, length, and/or atherosclerotic plaque burden, as well as in the take-off angle at which a renal artery branches from the aorta. Apparatus, systems and methods for achieving renal neuromodulation via intravascular access should account for these and other aspects of renal arterial anatomy and its variation across the patient population when minimally invasively accessing a renal artery.

In addition to complicating renal arterial access, specifics of the renal anatomy also complicate establishment of stable contact between neuromodulatory apparatus and a luminal surface or wall of a renal artery. When the neuromodulatory apparatus includes an energy delivery element, such as an electrode, consistent positioning and appropriate contact force applied by the energy delivery element to the vessel wall are important for predictability. However, navigation is impeded by the tight space within a renal artery, as well as tortuosity of the artery. Furthermore, establishing consistent contact is complicated by patient movement, respiration, and/or the cardiac cycle because these factors may cause significant movement of the renal artery relative to the aorta, and the cardiac cycle may transiently distend the renal artery (i.e. cause the wall of the artery to pulse.

Even after accessing a renal artery and facilitating stable contact between neuromodulatory apparatus and a luminal surface of the artery, nerves in and around the adventia of the artery should be safely modulated via the neuromodulatory apparatus. Effectively applying thermal treatment from within a renal artery is non-trivial given the potential clinical complications associated with such treatment. For example, the intima and media of the renal artery are highly vulnerable to thermal injury. As discussed in greater detail below, the intima-media thickness separating the vessel lumen from its adventitia means that target renal nerves may be multiple millimeters distant from the luminal surface of the artery. Sufficient energy should be delivered to or heat removed from the target renal nerves to modulate the target renal nerves without excessively cooling or heating the vessel wall to the extent that the wall is frozen, desiccated, or otherwise potentially effected to an undesirable extent. A potential clinical complication associated with excessive heating is thrombus formation from coagulating blood flowing through the artery. Given that this thrombus may cause a kidney infarct, thereby causing irreversible damage to the kidney, thermal treatment from within the renal artery should be applied carefully. Accordingly, the complex fluid mechanics and thermodynamic conditions present in the renal artery during treatment, particularly those that may impact heat transfer dynamics at the treatment site, may be important in applying energy (e.g., heating thermal energy) and/or removing heat from the tissue (e.g., cooling thermal conditions) from within the renal artery.

The neuromodulatory apparatus should also be configured to allow for adjustable positioning and repositioning of the energy delivery element within the renal artery since location of treatment may also impact clinical efficacy. For example, it may be tempting to apply a full circumferential treatment from within the renal artery given that the renal nerves may be spaced circumferentially around a renal artery. In some situations, full-circle lesion likely resulting from a continuous circumferential treatment may be potentially related to renal artery stenosis. Therefore, the formation of more complex lesions along a longitudinal dimension of the renal artery via the mesh structures described herein and/or repositioning of the neuromodulatory apparatus to multiple treatment locations may be desirable. It should be noted, however, that a benefit of creating a circumferential ablation may outweigh the potential of renal artery stenosis or the risk may be mitigated with certain embodiments or in certain patients and creating a circumferential ablation could be a goal. Additionally, variable positioning and repositioning of the neuromodulatory apparatus may prove to be useful in circumstances where the renal artery is particularly tortuous or where there are proximal branch vessels off the renal artery main vessel, making treatment in certain locations challenging. Manipulation of a device in a renal artery should also consider mechanical injury imposed by the device on the renal artery. Motion of a device in an artery, for example by inserting, manipulating, negotiating bends and so forth, may contribute to dissection, perforation, denuding intima, or disrupting the interior elastic lamina.

Blood flow through a renal artery may be temporarily occluded for a short time with minimal or no complications. However, occlusion for a significant amount of time should be avoided because to prevent injury to the kidney such as ischemia. It could be beneficial to avoid occlusion all together or, if occlusion is beneficial to the embodiment, to limit the duration of occlusion, for example to 2-5 minutes.

Based on the above described challenges of (1) renal artery intervention, (2) consistent and stable placement of the treatment element against the vessel wall, (3) effective application of treatment across the vessel wall, (4) positioning and potentially repositioning the treatment apparatus to allow for multiple treatment locations, and (5) avoiding or limiting duration of blood flow occlusion, various independent and dependent properties of the renal vasculature that may be of interest include, for example, (a) vessel diameter, vessel length, intima-media thickness, coefficient of friction, and tortuosity; (b) distensibility, stiffness and modulus of elasticity of the vessel wall; (c) peak systolic, end-diastolic blood flow velocity, as well as the mean systolic-diastolic peak blood flow velocity, and mean/max volumetric blood flow rate; (d) specific heat capacity of blood and/or of the vessel wall, thermal conductivity of blood and/or of the vessel wall, and/or thermal convectivity of blood flow past a vessel wall treatment site and/or radiative heat transfer; (e) renal artery motion relative to the aorta induced by respiration, patient movement, and/or blood flow pulsatility: and (f) as well as the take-off angle of a renal artery relative to the aorta. These properties will be discussed in greater detail with respect to the renal arteries. However, dependent on the apparatus, systems and methods utilized to achieve renal neuromodulation, such properties of the renal arteries, also may guide and/or constrain design characteristics.

As noted above, an apparatus positioned within a renal artery should conform to the geometry of the artery. Renal artery vessel diameter, D_{RA}, typically is in a range of about 2-10 mm, with most of the patient population having a D_{RA} of about 4mm to about 8mm and an average of about 6 mm. Renal artery vessel length, L_{RA}, between its ostium at the aorta/renal artery juncture and its distal branchings, generally is in a range of about 5-70 mm, and a significant portion of the patient population is in a range of about 20-50mm. Since the target renal plexus is embedded within the adventitia of the renal artery, the composite Intima-Media Thickness, IMT, (i.e., the radial outward distance from the artery's luminal surface to the adventitia containing target neural structures) also is notable and generally is in a range of about 0.5-2.5 mm, with an average of about 1.5 mm. Although a certain depth of treatment is important to reach the target neural fibers, the treatment should not be too deep (e.g., > 5 mm from inner wall of the renal artery) to avoid non-target tissue and anatomical structures such as the renal vein.

An additional property of the renal artery that may be of interest is the degree of renal motion relative to the aorta, induced by respiration and/or blood flow pulsatility. A patient's kidney, which located at the distal end of the renal artery, may move as much as 4" cranially with respiratory excursion. This may impart significant motion to the renal artery connecting the aorta and the kidney, thereby requiring from the neuromodulatory apparatus a unique balance of stiffness and flexibility to maintain contact between the thermal treatment element and the vessel wall during cycles of respiration. Furthermore, the take-off angle between the renal artery and the aorta may vary significantly between patients, and also may vary dynamically within a patient, e.g., due to kidney motion. The take-off angle generally may be in a range of about 30°-135°.

### VII. Conclusion

The above detailed descriptions of embodiments of the technology are not intended to be exhaustive or to limit the technology to the precise form disclosed above. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively. For example, much of the disclosure herein describes an energy delivery element 24 (e.g., an electrode) in the singular. It should be understood that this application does not exclude two or more energy delivery elements or electrodes.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

## Claims

1. A catheter apparatus for treatment of a human patient via renal denervation, the catheter apparatus comprising:
an elongated shaft (16) having a proximal portion (18) and a distal portion (20), wherein the distal portion (20) of the shaft (16) is configured for intravascular delivery to a renal artery of the patient;
a braided or woven mesh structure (22) at the distal portion (20) of the elongated shaft (16),
wherein the mesh structure includes a proximal portion and a distal portion, and wherein at least one of the proximal portion or the distal portion of the mesh structure is coupled to the elongated shaft via generally flexible wires (104); and
wherein the mesh structure (22) is configured to vary between a delivery configuration and a deployed configuration, and wherein the mesh structure (22) comprises interstitial spaces that allow blood to flow through the mesh structure (22) when in the deployed configuration; and
an energy delivery element (24) associated with the mesh structure (22), wherein the energy delivery element (24) is configured to thermally inhibit neural communication along the renal artery, and wherein, in the deployed configuration, the mesh structure (22) is configured to contact a wall of the renal artery and position the energy delivery element (24) in stable contact with the renal artery wall.

2. The catheter apparatus of claim 1, wherein the mesh structure (22) comprises a conformable tube, and wherein the interstitial spaces comprise holes or cutouts in the conformable tube, and/or wherein the mesh structure (22) comprises an insulating material configured to insulate against energy delivered by the energy delivery element (24), and/or wherein the mesh structure (22) comprises an electrically conductive material, and wherein the electrically conductive material comprises a portion of the energy delivery element (24), wherein the electrically conductive material is typically covered only in part by an electrically insulating material.

3. The catheter apparatus of claim 1, wherein:
the proximal portion of the mesh structure (22) is coupled to the distal portion of the elongated shaft (16); and
the distal portion of the mesh structure (22) is coupled to a second shaft, for example a second shaft (74) comprising a lumen or receptacle configured to receive a distal portion of a guide wire (66), and wherein the mesh structure (22) is configured to vary between the delivery configuration and the deployed configuration upon relative motion of the elongated shaft (16) and the second shaft (74).

4. The catheter apparatus of claim 1, wherein the elongated shaft (16) comprises a lumen configured to receive a guide wire (66), and/or wherein the catheter apparatus further comprised a sheath or guide catheter configured to deliver the mesh structure (22) into the renal artery and/or wherein the elongated shaft (16), the mesh structure (22), and the energy delivery element (24) are configured for intravascular delivery into the renal artery via a 6 French or smaller guide catheter.

5. The catheter apparatus of claim 1, further comprising a pull wire (68) coupled to the mesh structure (22) and configured to actuate the mesh structure (22) between the deployed configuration and the delivery configuration, for example the catheter apparatus further comprises a handle (34) coupled to the proximal end of the elongated shaft (16), wherein the handle (34) comprises an actuatable element (36) configured to adjust tension in the pull wire (68).

6. The cathether apparatus of claim 1, wherein the mesh structure (22) comprises one or more heat-set mesh points (2881) or protrusions (2981), and/or wherein the mesh structure (22) comprises one or more annular mesh rings, and/or wherein the mesh structure (22) comprises a radiopaque material.

7. The catheter apparatus of claim 1, wherein the energy delivery element (24) is configured to apply at least one of radiofrequency energy, microwave energy, ultrasound energy, laser energy, electromagnetic energy, or thermal energy to the renal artery.

8. The catheter apparatus of claim 1, further comprising a sensor associated with the energy delivery element (24), wherein the sensor is configured to monitor a parameter of at least one of the apparatus, blood, or the renal artery.

9. The catheter apparatus of claim 1, wherein the at least one energy delivery element (24) comprises two energy delivery elements that are spaced apart from each other along a longitudinal axis of the elongated shaft (16) and circumferentially offset along the circumference of the mesh structure (22), for example the two energy delivery elements (24) are at least 5 mm apart from one another along the longitudinal axis of the elongated shaft (16) when the mesh structure (22) is in a fully deployed configuration.

10. The catheter apparatus of claim 1, wherein a length of the mesh structure (22) relative to the elongated shaft (16) is less when the mesh structure (22) is in the deployed configuration relative to when the mesh structure (22) is in the delivery configuration, for example the mesh structure (22) has a length in a fully deployed configuration from about 50% to about 80% of a length of the mesh structure (22) in the delivery configuration, for example a length of the mesh structure (22) when in a fully deployed configuration is less than about 30 mm and the length of the mesh structure (22) when in the delivery configuration is less than about 40 mm.

11. The catheter apparatus of claim 1, wherein the mesh structure (22) is mounted to the distal end of the elongated shaft (16), and/or wherein the mesh structure (22) is located distally from the distal end of the elongated shaft (16), and/or wherein the mesh structure (22) in the deployed configuration is configured to apply a radial force to the renal artery wall, for example the radial force is no more than about 300 mN/mm, and/or wherein a largest diameter of the mesh structure (22) in a fully deployed configuration is from about 8 mm to about 10 mm or wherein the mesh structure (22) in a fully deployed configuration has a largest diameter from about 5 mm to about 8 mm, or wherein the mesh structure (22) in a fully deployed configuration has a largest diameter from about 3 mm to about 5 mm.

12. The catheter apparatus of claim 1, wherein the mesh structure (22) has a diameter no greater than about 10 mm while in the fully deployed configuration;
wherein when the mesh structure (22) is deployed within the renal artery to a diameter that is less than the largest diameter of the fully deployed configuration, the mesh structure (22) is configured to contact an inner wall of the renal artery and position the energy delivery element (24) in stable contact with the inner wall.

13. The catheter apparatus of claim 12, wherein the mesh structure (22) comprises a conformable tube having interstitial spaces comprising holes or cutouts in the conformable tube, or wherein the mesh structure (22) includes a proximal portion and a distal portion, and wherein the mesh structure (16) is coupled to the elongated shaft (16) at only one of the proximal or distal portions of the mesh structure (22), for example the proximal portion of the mesh structure (22) is coupled to the distal portion (20) of the elongated shaft (16), or wherein, for example, the distal portion of the mesh structure (22) is coupled to a wire extending from the distal portion of the elongated shaft (16), and wherein the proximal portion of the mesh structure (22) is not coupled to the elongated shaft (16).

14. The catheter apparatus of claim 12, wherein the largest diameter of the fully expanded configuration is from about 8 mm to about 10 mm, and wherein the diameter that is less than the largest diameter of the fully expanded configuration is about 6 mm or less.

15. The catheter apparatus of claim 12, wherein the at least one energy delivery element (24) comprises a plurality of energy delivery elements, and wherein each individual energy delivery element is electrically connected to the other energy delivery elements, or wherein each individual energy delivery element is electrically isolated from the other energy delivery elements.

## Patentansprüche

1. Kathetervorrichtung zur Behandlung eines menschlichen Patienten über renale Denervierung, wobei die Kathetervorrichtung Folgendes umfasst:
einen länglichen Schaft (16) mit einem proximalen Abschnitt (18) und einem distalen Abschnitt (20), wobei der distale Abschnitt (20) des Schafts (16) für eine intravaskuläre Abgabe an eine Nierenarterie des Patienten konfiguriert ist;
eine geflochtene oder gewebte Netzstruktur (22) an dem distalen Abschnitt (20) des länglichen Schafts (16), wobei die Netzstruktur einen proximalen Abschnitt und einen distalen Abschnitt beinhaltet und wobei mindestens einer von dem proximalen Abschnitt oder dem distalen Abschnitt der Netzstruktur mit dem länglichen Schaft über im Allgemeinen flexible Drähte (104) gekoppelt ist; und
wobei die Netzstruktur (22) dazu konfiguriert ist, zwischen einer Abgabekonfiguration und einer entfalteten Konfiguration zu variieren, und wobei die Netzstruktur (22) Zwischenräume umfasst, die zulassen, dass Blut in der entfalteten Konfiguration durch die Netzstruktur (22) fließt; und
ein Energieabgabeelement (24), das mit der Netzstruktur (22) assoziiert ist, wobei das Energieabgabeelement (24) dazu konfiguriert ist, eine neuronale Kommunikation entlang der Nierenarterie thermisch zu unterbinden, und wobei in der entfalteten Konfiguration die Netzstruktur (22) dazu konfiguriert ist, mit einer Wand der Nierenarterie in Kontakt zu treten und das Energieabgabeelement (24) in einem stabilen Kontakt mit der Nierenarterienwand zu positionieren.

2. Kathetervorrichtung nach Anspruch 1, wobei die Netzstruktur (22) ein anpassungsfähiges Rohr umfasst, und wobei die Zwischenräume Löcher oder Ausschnitte in dem anpassungsfähigen Rohr umfassen und/oder wobei die Netzstruktur (22) ein Isoliermaterial umfasst, das dazu konfiguriert ist, gegen von dem Energieabgabeelement (24) abgegebene Energie zu isolieren, und/oder wobei die Netzstruktur (22) ein elektrisch leitfähiges Material umfasst, und wobei das elektrisch leitfähige Material einen Abschnitt des Energieabgabeelements (24) umfasst, wobei das elektrisch leitfähige Material typischerweise nur teilweise von einem elektrischen Isoliermaterial überdeckt ist.

3. Kathetervorrichtung nach Anspruch 1, wobei:
der proximale Abschnitt der Netzstruktur (22) mit dem distalen Abschnitt des länglichen Schafts (16) gekoppelt ist; und
der distale Abschnitt der Netzstruktur (22) mit einem zweiten Schaft gekoppelt ist, zum Beispiel einem zweiten Schaft (74), der ein Lumen oder eine Aufnahme umfasst, das bzw. die dazu konfiguriert ist, einen distalen Abschnitt eines Führungsdrahts (66) aufzunehmen, und wobei die Netzstruktur (22) dazu konfiguriert ist, zwischen der Abgabekonfiguration und der entfalteten Konfiguration bei einer relativen Bewegung des länglichen Schafts (16) und des zweiten Schafts (74) zu variieren.

4. Kathetervorrichtung nach Anspruch 1, wobei der längliche Schaft (16) ein Lumen umfasst, das dazu konfiguriert ist, einen Führungsdraht (66) aufzunehmen und/oder wobei die Kathetervorrichtung ferner eine Hülle oder einen Führungskatheter umfasst, die bzw. der konfiguriert ist, die Netzstruktur (22) in die Nierenarterie abzugeben und/oder wobei der längliche Schaft (16), die Netzstruktur (22) und das Energieabgabeelement (24) zur intravaskulären Abgabe in die Nierenarterie über einen 6 French oder kleineren Führungskatheter konfiguriert sind.

5. Kathetervorrichtung nach Anspruch 1, ferner umfassend einen Zugdraht (68), der mit der Netzstruktur (22) gekoppelt ist und dazu konfiguriert ist, die Netzstruktur (22) zwischen der entfalteten Konfiguration und der Abgabekonfiguration zu betätigen, wobei die Kathetervorrichtung zum Beispiel ferner einen Handgriff (34) umfasst, der mit dem proximalen Ende des länglichen Schafts (16) gekoppelt ist, wobei der Handgriff (34) ein betätigbares Element (36) umfasst, das dazu konfiguriert ist, eine Spannung im Zugdraht einzustellen (68).

6. Kathetervorrichtung nach Anspruch 1, wobei die Netzstruktur (22) einen oder mehrere thermofixierte Netzpunkte (2881) oder Vorsprünge (2981) umfasst, und/oder wobei die Netzstruktur (22) einen oder mehrere ringförmige Netzringe umfasst, und/oder wobei die Netzstruktur (22) ein strahlenundurchlässiges Material umfasst.

7. Kathetervorrichtung nach Anspruch 1, wobei das Energieabgabeelement (24) dazu konfiguriert ist, mindestens eine von Hochfrequenzenergie, Mikrowellenenergie, Ultraschallenergie, Laserenergie, elektromagnetischer Energie oder Wärmeenergie an der Nierenarterie aufzubringen.

8. Kathetervorrichtung nach Anspruch 1, ferner umfassend einen mit dem Energieabgabeelement (24) assoziierten Sensor, wobei der Sensor dazu konfiguriert ist, einen Parameter von mindestens einem von der Vorrichtung, Blut oder der Nierenarterie zu überwachen.

9. Kathetervorrichtung nach Anspruch 1, wobei das mindestens eine Energieabgabeelement (24) zwei Energieabgabeelemente umfasst, die entlang einer Längsachse des länglichen Schafts (16) voneinander beabstandet und in Umfangsrichtung entlang des Umfangs der Netzstruktur (22) versetzt sind, zum Beispiel sind die beiden Energieabgabeelemente (24) entlang der Längsachse des länglichen Schafts (16) mindestens 5 mm voneinander entfernt, wenn sich die Netzstruktur (22) in einer vollständig entfalteten Konfiguration befindet.

10. Kathetervorrichtung nach Anspruch 1, wobei eine Länge der Netzstruktur (22) in Bezug auf den länglichen Schaft (16) kleiner ist, wenn sich die Netzstruktur (22) in der entfalteten Konfiguration befindet, als wenn sich die Netzstruktur (22) in der Abgabekonfiguration befindet, beispielweise weist die Netzstruktur (22) eine Länge in einer vollständig entfalteten Konfiguration von etwa 50 % bis etwa 80 % einer Länge der Netzstruktur (22) in der Abgabekonfiguration auf, zum Beispiel beträgt eine Länge der Netzstruktur (22) in einer vollständig entfalteten Konfiguration weniger als etwa 30 mm und beträgt die Länge der Netzstruktur (22) in der Abgabekonfiguration weniger als etwa 40 mm.

11. Kathetervorrichtung nach Anspruch 1, wobei die Netzstruktur (22) am distalen Ende des länglichen Schafts (16) angebracht ist und/oder wobei sich die Netzstruktur (22) distal vom distalen Ende des länglichen Schafts (16) befindet und/oder wobei die Netzstruktur (22) in der entfalteten Konfiguration dazu konfiguriert ist, eine Radialkraft auf die Nierenarterienwand aufzubringen, zum Beispiel beträgt die Radialkraft nicht mehr als etwa 300 mN/mm, und/oder wobei ein größter Durchmesser der Netzstruktur (22) in einer vollständig entfalteten Konfiguration von etwa 8 mm bis etwa 10 mm beträgt oder wobei die Netzstruktur (22) in einer vollständig entfalteten Konfiguration einen größten Durchmesser von etwa 5 mm bis etwa 8 mm aufweist, oder wobei die Netzstruktur (22) in einer vollständig entfalteten Konfiguration einen größten Durchmesser von etwa 3 mm bis etwa 5 mm aufweist.

12. Kathetervorrichtung nach Anspruch 1, wobei die Netzstruktur (22) in der vollständig entfalteten Konfiguration einen Durchmesser nicht größer als etwa 10 mm aufweist;
wobei, wenn die Netzstruktur (22) innerhalb der Nierenarterie bis zu einem Durchmesser entfaltet ist, der kleiner als der größte Durchmesser der vollständig entfalteten Konfiguration ist, die Netzstruktur (22) dazu konfiguriert ist, mit einer Innenwand der Nierenarterie in Kontakt zu treten und das Energieabgabeelement (24) in stabilem Kontakt mit der Innenwand zu positionieren.

13. Kathetervorrichtung nach Anspruch 12, wobei die Netzstruktur (22) ein anpassungsfähiges Rohr mit Zwischenräumen umfasst, die Löcher oder Ausschnitte in dem anpassungsfähigen Rohr umfassen oder wobei die Netzstruktur (22) einen proximalen Abschnitt und einen distalen Abschnitt beinhaltet, und wobei die Netzstruktur (16) mit dem länglichen Schaft (16) nur an einem von dem proximalen oder distalen Abschnitt der Netzstruktur (22) gekoppelt ist, zum Beispiel ist der proximale Abschnitt der Netzstruktur (22) mit dem distalen Abschnitt (20) des länglichen Schafts (16) gekoppelt, oder wobei zum Beispiel der distale Abschnitt der Netzstruktur (22) mit einem Draht gekoppelt ist, der sich von dem distalen Abschnitt des länglichen Schafts (16) erstreckt und wobei der proximale Abschnitt der Netzstruktur (22) nicht mit dem länglichen Schaft (16) gekoppelt ist.

14. Kathetervorrichtung nach Anspruch 12, wobei der größte Durchmesser der vollständig expandierten Konfiguration von etwa 8 mm bis etwa 10 mm beträgt, und wobei der Durchmesser, der kleiner als der größte Durchmesser der vollständig expandierten Konfiguration ist, etwa 6 mm oder weniger beträgt.

15. Kathetervorrichtung nach Anspruch 12, wobei das mindestens eine Energieabgabeelement (24) eine Vielzahl von Energieabgabeelementen umfasst, und wobei jedes einzelne Energieabgabeelement mit den anderen Energieabgabeelementen elektrisch verbunden ist, oder wobei jedes einzelne Energieabgabeelement von der anderen Energieabgabeelementen elektrisch isoliert ist.

## Revendications

1. Appareil de cathéter pour le traitement d'un patient humain par dénervation rénale, l'appareil de cathéter comprenant :
une tige allongée (16) ayant une partie proximale (18) et une partie distale (20), dans lequel la partie distale (20) de la tige (16) est configurée pour une administration intravasculaire à une artère rénale du patient ;
une structure de maille tressée ou tissée (22) au niveau de la partie distale (20) de la tige allongée (16),
dans lequel la structure de maille comporte une partie proximale et une partie distale, et dans lequel au moins l'une de la partie proximale ou de la partie distale de la structure de maille est couplée à la tige allongée par des fils généralement souples (104) ; et
dans lequel la structure de maille (22) est configurée pour varier entre une configuration d'administration et une configuration déployée, et dans lequel la structure de maille (22) comprend des espaces interstitiels qui permettent au sang de circuler à travers la structure de maille (22) lorsqu'elle est dans la configuration déployée ; et
un élément d'administration d'énergie (24) associé à la structure de maille (22), dans lequel l'élément d'administration d'énergie (24) est configuré pour inhiber thermiquement la communication neuronale le long de l'artère rénale, et dans lequel, dans la configuration déployée, la structure de maille (22) est configurée pour entrer en contact avec une paroi de l'artère rénale et positionner l'élément d'administration d'énergie (24) en contact stable avec la paroi d'artère rénale.

2. Appareil de cathéter selon la revendication 1, dans lequel la structure de maille (22) comprend un tube souple, et dans lequel les espaces interstitiels comprennent des trous ou des découpes dans le tube souple, et/ou dans lequel la structure de maille (22) comprend un matériau isolant configuré pour isoler contre l'énergie administrée par l'élément d'administration d'énergie (24), et/ou dans lequel la structure de maille (22) comprend un matériau électriquement conducteur, et dans lequel le matériau électriquement conducteur comprend une partie de l'élément d'administration d'énergie (24), dans lequel le matériau électriquement conducteur est généralement recouvert uniquement en partie par un matériau électriquement isolant.

3. Appareil de cathéter selon la revendication 1, dans lequel :
la partie proximale de la structure de maille (22) est couplée à la partie distale de la tige allongée (16) ; et
la partie distale de la structure de maille (22) est couplée à une seconde tige, par exemple, une seconde tige (74) comprenant une lumière ou un réceptacle configurés pour recevoir une partie distale d'un fil de guidage (66), et dans lequel la structure de maille (22) est configurée pour varier entre la configuration d'administration et la configuration déployée lors du mouvement relatif de la tige allongée (16) et de la seconde tige (74).

4. Appareil de cathéter selon la revendication 1, dans lequel la tige allongée (16) comprend une lumière configurée pour recevoir un fil de guidage (66), et/ou dans lequel l'appareil de cathéter comprend en outre un cathéter à gaine ou de guidage configuré pour administrer la structure de maille (22) dans l'artère rénale et/ou dans lequel la tige allongée (16), la structure de maille (22) et l'élément d'administration d'énergie (24) sont configurés pour une administration intravasculaire dans l'artère rénale par un cathéter français 6 ou un cathéter de guidage plus petit.

5. Appareil de cathéter selon la revendication 1, comprenant en outre un fil de traction (68) couplé à la structure de maille (22) et configuré pour actionner la structure de maille (22) entre la configuration déployée et la configuration d'administration, par exemple, l'appareil de cathéter comprend en outre une poignée (34) couplée à l'extrémité proximale de la tige allongée (16), dans lequel la poignée (34) comprend un élément pouvant être actionné (36) configuré pour ajuster la tension dans le fil de traction (68).

6. Appareil de cathéter selon la revendication 1, dans lequel la structure de maille (22) comprend un ou plusieurs points de maillage thermofixés (2881) ou saillies (2981), et/ou dans lequel la structure de maille (22) comprend une ou plusieurs bagues en matière à mailles annulaires et/ou dans lequel la structure de maille (22) comprend un matériau radio-opaque.

7. Appareil de cathéter selon la revendication 1, dans lequel l'élément d'administration d'énergie (24) est configuré pour appliquer au moins l'une parmi l'énergie radiofréquence, l'énergie hyperfréquence, l'énergie ultrasonore, l'énergie laser, l'énergie électromagnétique ou l'énergie thermique à l'artère rénale.

8. Appareil de cathéter selon la revendication 1, comprenant en outre un capteur associé à l'élément d'administration d'énergie (24), dans lequel le capteur est configuré pour surveiller un paramètre d'au moins l'un parmi l'appareil, le sang ou l'artère rénale.

9. Appareil de cathéter selon la revendication 1, dans lequel l'au moins un élément d'administration d'énergie (24) comprend deux éléments d'administration d'énergie qui sont espacés l'un de l'autre le long d'un axe longitudinal de la tige allongée (16) et décalés circonférentiellement le long de la circonférence de la structure de maille (22), par exemple, les deux éléments d'administration d'énergie (24) sont espacés d'au moins 5 mm l'un de l'autre le long de l'axe longitudinal de la tige allongée (16) lorsque la structure de maille (22) est dans une configuration complètement déployée.

10. Appareil de cathéter selon la revendication 1, dans lequel une longueur de la structure de maille (22) par rapport à la tige allongée (16) est inférieure lorsque la structure de maille (22) est dans la configuration déployée par rapport à lorsque la structure de maille (22) est dans la configuration d'administration, par exemple, la structure de maille (22) a une longueur dans une configuration complètement déployée d'environ 50 % à environ 80 % d'une longueur de la structure de maille (22) dans la configuration d'administration, par exemple, une longueur de la structure de maille (22) lorsqu'elle est dans une configuration complètement déployée est inférieure à environ 30 mm et la longueur de la structure de maille (22) lorsqu'elle est dans la configuration d'administration est inférieure à environ 40 mm.

11. Appareil de cathéter selon la revendication 1, dans lequel la structure de maille (22) est montée sur l'extrémité distale de la tige allongée (16), et/ou dans lequel la structure de maille (22) est située de manière distale depuis l'extrémité distale de la tige allongée (16), et/ou dans lequel la structure de maille (22) dans la configuration déployée est configurée pour appliquer une force radiale à la paroi d'artère rénale, par exemple, la force radiale ne dépasse pas environ 300 mN/mm, et/ou dans lequel un diamètre le plus grand de la structure de maille (22) dans une configuration complètement déployée est d'environ 8 mm à environ 10 mm ou dans lequel la structure de maille (22) dans une configuration complètement déployée a un diamètre le plus grand d'environ 5 mm à environ 8 mm, ou dans lequel la structure de maille (22) dans une configuration complètement déployée a un diamètre le plus grand d'environ 3 mm à environ 5 mm.

12. Appareil de cathéter selon la revendication 1, dans lequel la structure de maille (22) a un diamètre non supérieur à environ 10 mm lorsqu'elle est dans la configuration complètement déployée ;
dans lequel, lorsque la structure de maille (22) est déployée à l'intérieur de l'artère rénale jusqu'à un diamètre qui est inférieur au diamètre le plus grand de la configuration complètement déployée, la structure de maille (22) est configurée pour entrer en contact avec une paroi interne de l'artère rénale et positionner l'élément d'administration d'énergie (24) en contact stable avec la paroi interne.

13. Appareil de cathéter selon la revendication 12, dans lequel la structure de maille (22) comprend un tube souple ayant des espaces interstitiels comprenant des trous ou des découpes dans le tube souple, ou dans lequel la structure de maille (22) comporte une partie proximale et une partie distale, et dans lequel la structure de maille (16) est couplée à la tige allongée (16) au niveau d'une seule des parties proximale ou distale de la structure de maille (22), par exemple, la partie proximale de la structure de maille (22) est couplée à la partie distale (20) de la tige allongée (16), ou dans lequel, par exemple, la partie distale de la structure de maille (22) est couplée à un fil s'étendant depuis la partie distale de la tige allongée (16), et dans lequel la partie proximale de la structure de maille (22) n'est pas couplée à la tige allongée (16).

14. Appareil de cathéter selon la revendication 12, dans lequel le diamètre le plus grand de la configuration complètement dilatée est d'environ 8 mm à environ 10 mm, et dans lequel le diamètre qui est inférieur au diamètre le plus grand de la configuration complètement dilatée est d'environ 6 mm ou moins.

15. Appareil de cathéter selon la revendication 12, dans lequel l'au moins un élément d'administration d'énergie (24) comprend une pluralité d'éléments d'administration d'énergie, et dans lequel chaque élément d'administration d'énergie individuel est relié électriquement aux autres éléments d'administration d'énergie, ou dans lequel chaque élément d'administration d'énergie individuel est isolé électriquement des autres éléments d'administration d'énergie.
